# EUROPEAN PATENT APPLICATION

(11) **EP 3 536 796 A1**
(43) Date of publication of application: **11.09.2019**
(21) Application number: 17812759.3
(22) Date of filing: 16.06.2017
(51) Int. Cl.: C12N 15/90, C12N 15/11

(54) **GENE KNOCKOUT METHOD**

(30) Priority: 17.06.2016 CN 201610439385
(71) Applicant: Edigene Biotechnology Inc., Beijing 102206 (CN)
(72) Inventor: WEI, Wensheng, Beijing 100871 (CN); ZHOU, Yuexin, Beijing 100871 (CN); ZHANG, Hongmin, Beijing 100871 (CN)
(74) Representative: Boult Wade Tennant LLP
(86) International application number: PCT/CN2017/088582
(87) International publication number: WO 2017/215648

(57) **Abstract**

Provided are a donor construct, a gene knockout method, and a system and a kit for gene knockout. In the gene knockout method, a marker gene contained in the donor construct is inserted into a double-strand break position in the cell genome by non-homologous end joining, thus improving the production efficiency of gene knockout by a sequence-specific nuclease through enrichment of gene knockout cells via the expressed marker.

## Description

### Technical Field

The present invention relates to the gene editing technology, and in particular to a gene knockout method.

### Background Art

The gene editing technology has revolutionized experimental research on gene functions. Three major technologies, ZFNs (zinc finger nucleases) ^{[1]}, TALENs (transcription activator-like effector nucleases) ^{[2-4]} and CRISPR/Cas9 systems ^{[5-7]}, employ different mechanisms to generate sequence-specific double-strand breaks (DSBs) and subsequently trigger natural repair systems to complete sequence-specific modifications ^{[8,9]}. These technologies have been widely used in functional gene research ^{[10]}, dynamic and real-time imaging of chromosomal loci ^{[11,12]}, correction of disease mutations ^{[13]}, gene therapy ^{[14]} and other aspects. The CRISPR/Cas9 systems are becoming particularly popular for its efficiency and ease of operation. The CRISPR/Cas9 systems were originally used by the bacterial immune system to fight off foreign viruses or plasmids. In the Class II CRISPR system, Cas9 endonucleases cleave double-stranded DNAs under the guidance of sgRNAs, resulting in double-strand breaks in the genome, and making use of the instability of cell genome repair to produce repair errors (deletion or insertion of bases), thus producing the effect of gene editing.

Although the CRISPR/Cas9 systems have unprecedented advantages in design- and sequence-specificity-based genomic research, gene editing triggered by the CRISPR/Cas9 systems remains a rare event in cell populations. It requires tedious labour to get real genetically edited single clones. Therefore, the systems are still technically challenging, even for the simple task of producing gene knockout in mammalian cells ^{[15]}. Various efforts have been made to improve the efficiency of the protocol of producing gene knockout, for instance, integrating the CRISPR/Cas9 systems to permanently express Cas9s and sgRNAs ^{[16]}, generating cell lines stably expressing Cas9 in advance ^{[17]}, enhancing non-homologous end joining (NHEJ) pathway ^{[18]}, enriching gene-targeted events by simultaneously destroying single genes that can achieve specific drug selection ^{[19]}, and enriching gene knockout by surrogate reporters ^{[20,21]}. However, various shortcomings limit the wide use of these technologies. In particular, it remains a difficult task to generate multi-gene knockouts in mammalian cells. When using traditional methods, it is sometimes a time-consuming, onerous and high-risk task even to knock out a single gene ^{[22]}, as these methods lack effective enrichment for rare clones containing target gene modifications.

If the destruction of the target gene can result in a phenotypic change that can be used for enrichment, gene knockout clones can be easily obtained, for instance, Hela *CSPG4*^{*-*/*-*} cells confer resistance to *Clostridium difficile* toxin B ^{[23]}. However, this strategy is not universal. The traditional method involves co-transfection of plasmids expressing antibiotic resistance or fluorescent proteins ^{[23,24]}, but this method does not enrich a small number of cells containing targeted modifications.

It has been reported that exogenous dsDNA fragments may be integrated into chromosomal loci with DSBs through different repair mechanisms. A long flanking sequence needs to be constructed through homologous recombination (HR) repair, and the integration efficiency is low; while the integration efficiency of Non-Homologous end joining (NHEJ) DNA repair ^{[27,28]} is usually higher than that of homologous recombination repair ^{[29]}. Previous studies have used NHEJ triggered by CRISPR/Cas to mediate insertion of exogenous linear donor DNAs to achieve the purpose of gene knockin ^{[30-34]}.

### Summary of the Invention

The present invention provides a donor construct and a gene knockout method, as well as a system and a kit for gene knockout. The gene knockout method of the present invention makes use of a marker gene contained in the donor construct to enrich gene knockout cells, thus improving the production efficiency of gene knockout by a sequence-specific nuclease.

According to one aspect of the present invention, a donor construct is provided which is a linear donor DNA or capable of producing a linear donor DNA by cleavage in a cell, wherein the linear donor DNA sequentially comprises, from the middle to both ends: an expression cassette; a short sequence extension consisting of reverse termination codons which is located at the 5'-end of the expression cassette and a short sequence extension consisting of forward termination codons which is located at the 3'-end of the expression cassette; a target sequence located at the 5'-end and/or 3'-end, the target sequence comprising a target site capable of being cleaved by a sequence-specific nuclease; and protection sequences located at both ends; and wherein the expression cassette comprises a promoter-driven marker gene.

In the present invention, the linear donor DNA is a double-stranded linear donor DNA.

In a preferred embodiment, the donor construct is a linear donor DNA.

In some embodiments, the sequence-specific nuclease is a zinc finger nuclease (ZFN).

In still some embodiments, the sequence-specific nuclease is a transcription activator-like effector nuclease (TALEN).

In still some embodiments, the sequence-specific nuclease is a Cas9 nuclease.

In still some embodiments, the sequence-specific nuclease is an NgAgo nuclease.

In some embodiments, the linear donor DNA has a target sequence only at the 5'-end or 3'-end.

In some embodiments, the linear donor DNA has target sequences at both ends, respectively.

In some embodiments, the target sequences at both ends of the linear donor DNA are the same.

In some embodiments, the target sequences at both ends of the linear donor DNA are different. In a further embodiment, the different target sequences at both ends of the linear donor DNA are derived from the same gene. In other further embodiments, the different target sequences at both ends of the linear donor DNA are derived from different genes.

In a preferred embodiment, the marker gene is an antibiotic resistance gene or a fluorescent protein gene.

In a preferred embodiment, the protection sequence is 5-30 bp, and most preferably 20 bp, in length.

According to another aspect of the present invention, a method of generating a gene knockout in a cell is provided, the method comprising the steps of:
(1) introducing into a cell a sequence-specific nuclease capable of cleaving a specific target site in the cell genome and a donor construct;
   wherein the donor construct is a linear donor DNA or capable of producing a linear donor DNA by cleavage in a cell, wherein the linear donor DNA sequentially comprises, from the middle to both ends: an expression cassette; a short sequence extension consisting of reverse termination codons which is located at the 5'-end of the expression cassette and a short sequence extension consisting of forward termination codons which is located at the 3'-end of the expression cassette; a target sequence located at the 5'-end and/or 3'-end, the target sequence comprising a target site capable of being cleaved by the sequence-specific nuclease; and protection sequences located at both ends; and wherein the expression cassette comprises a promoter-driven marker gene; and
   wherein the linear donor DNA is inserted into the specific target site in the cell genome by non-homologous end joining; and
(2) screening cells positive for the marker expression.

In the present invention, the linear donor DNA is a double-stranded linear donor DNA.

In a preferred embodiment, the donor construct is a linear donor DNA.

In some embodiments, the linear donor DNA has a target sequence only at the 5'-end or 3'-end.

In some embodiments, the linear donor DNA has target sequences at both ends, respectively.

In some embodiments, the target sequences at both ends of the linear donor DNA are the same.

In some embodiments, the target sequences at both ends of the linear donor DNA are different. In a further embodiment, the different target sequences at both ends of the linear donor DNA are derived from the same gene. In other further embodiments, the different target sequences at both ends of the linear donor DNA are derived from different genes.

In some embodiments, the sequence-specific nuclease is a zinc finger nuclease (ZFN).

In still some embodiments, the sequence-specific nuclease is a transcription activator-like effector nuclease (TALEN).

In still some embodiments, the sequence-specific nuclease is a Cas9 nuclease.

In a preferred embodiment, the method further comprises introducing into a cell a guide RNA (gRNA) that recognizes a specific target site in the cell genome, wherein the target sequence in the linear donor DNA comprises a target site recognized by the gRNA.

In some embodiments, the gRNA is an sgRNA.

In a more preferred embodiment, the method further comprises introducing into a cell an sgRNA that recognizes a single specific target site in the cell genome, wherein the target sequence comprising the target site recognized by the sgRNA is located at the 5'-end and/or 3'-end of the linear donor DNA. In some embodiments, the target sequence comprising the target site recognized by the sgRNA is derived from a single gene in the cell genome. In some embodiments, the target sequence comprising the target site recognized by the sgRNA is the consensus sequence of two or more genes in the cell genome, provided that the consensus sequence has no more than one base difference from the sequence at positions corresponding to the consensus sequence in any of the two or more genes.

In still some more preferred embodiments, the method further comprises introducing into a cell two sgRNAs that recognize two specific target sites on one gene in the cell genome, wherein two target sequences respectively comprising the two target sites recognized by the two sgRNAs are located in two linear donor DNAs, respectively, or located at both ends of the same linear donor DNA, respectively.

In still some more preferred embodiments, the method further comprises introducing into a cell two or more sgRNAs that recognize two or more specific target sites in the cell genome, wherein two or more target sequences respectively comprising the two or more target sites recognized by the two or more sgRNAs are located at both ends of the same linear donor DNA, respectively, or located in different linear donor DNAs. The two or more specific target sites in the cell genome are located on different genes, respectively.

In still some embodiments, the sequence-specific nuclease is an NgAgo nuclease.

In a preferred embodiment, the method further comprises introducing into a cell a guide DNA (gDNA) that recognizes a specific target site in the cell genome, wherein the target sequence in the linear donor DNA comprises a target site recognized by the gDNA.

In the present invention, the gene knockout can be a single gene knockout or a multi-gene knockout. The multi-gene knockout is knockout of two or more genes, such as knockout of three, four, five or more genes.

In a preferred embodiment, the marker gene is an antibiotic resistance gene or a fluorescent protein gene.

In a preferred embodiment, the cells are screened by drug resistance.

In another preferred embodiment, the cells are screened by the FACS method.

In a preferred embodiment, the protection sequence is 5-30 bp, and most preferably 20 bp, in length.

According to another aspect of the present invention, a system or a kit for gene knockout is provided, comprising: a sequence-specific nuclease capable of cleaving a specific target site in the cell genome and a donor construct;
wherein the donor construct is a linear donor DNA or capable of producing a linear donor DNA by cleavage in a cell, wherein the linear donor DNA sequentially comprises, from the middle to both ends: an expression cassette; a short sequence extension consisting of reverse termination codons which is located at the 5'-end of the expression cassette and a short sequence extension consisting of forward termination codons which is located at the 3'-end of the expression cassette; a target sequence located at the 5'-end and/or 3'-end, the target sequence comprising a target site capable of being cleaved by a sequence-specific nuclease; and protection sequences located at both ends; and wherein the expression cassette comprises a promoter-driven marker gene.

In the present invention, the linear donor DNA is a double-stranded linear donor DNA.

In some embodiments, the donor construct is a linear donor DNA. In still some embodiments, the donor construct is a circular donor construct and is capable of producing a linear donor DNA by cleavage in a cell.

In some embodiments, the sequence-specific nuclease is a zinc finger nuclease (ZFN).

In still some embodiments, the sequence-specific nuclease is a transcription activator-like effector nuclease (TALEN).

In still some embodiments, the sequence-specific nuclease is a Cas9 nuclease.

In a preferred embodiment, the system or kit further comprises an sgRNA that recognizes a specific target site in the cell genome, wherein the target sequence in the linear donor DNA comprises a target site recognized by the sgRNA.

In some embodiments, the gRNA is an sgRNA.

In still some embodiments, the sequence-specific nuclease is an NgAgo nuclease.

In a preferred embodiment, the system or kit further comprises a gDNA that recognizes a specific target site in the cell genome, wherein the target sequence in the linear donor DNA comprises a target site recognized by the gDNA.

In a preferred embodiment, the marker gene is an antibiotic resistance gene or a fluorescent protein gene.

In a preferred embodiment, the protection sequence is 5-30 bp, and most preferably 20 bp, in length.

In the present invention, the cleavage is to generate double-strand breaks (DSBs).

In the present invention, by inserting a marker gene into a cleavage target site for gene knockout, rare clones with the generated gene knockout can be effectively enriched through the marker. The present invention is particularly useful for targeting genes that are difficult to design sgRNAs, and in cases where several gene knockouts need to be targeted simultaneously. This method is useful for various gene editing systems that produce DNA double-strand breaks, especially for the wider use of the CRISPR systems in the biomedical field of genes and their functions.

### Brief Description of the Drawings

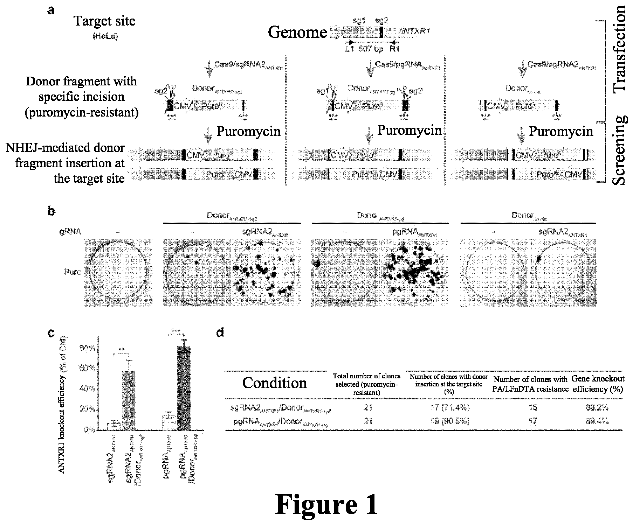

Figure 1 shows the donor design and experimental verification of enrichment of cells containing Cas9/gRNA-targeting mutations in the *ANTXR1* gene in HeLa cells by puromycin selection. (a) Schematic diagram of knockin of linear donor at sgRNA- or pgRNA-targeting site in *ANTXR1* gene based on NHEJ. The genomic locus and linear donor have cleavage sites sgl and sg2 for the gRNA. The termination codon is marked with ***, and the arrow points in the direction of the open reading frame. (b) MTT staining of puromycin-resistant clones of cells transfected with a donor (with or without gRNA). (c) Comparison of *ANTXR1* knockout rates of HeLa cells transfected with sgRNA/pgRNA (with (a dark stripe) or without (a light stripe) their corresponding donors). The *ANTXR1* knockout rate is expressed as the percentage of cells resistant to PA/LFnDTA. Cells were selected with puromycin (1 µg/ml) prior to PA/LFnDTA resistance analysis. Error line indicates s.d. (n = 3), *t*-test, ***P*< 0.01, ****P<* 0.001. (d) Summary of *ANTXR1* knockout cells enriched via different gRNAs and their donors.

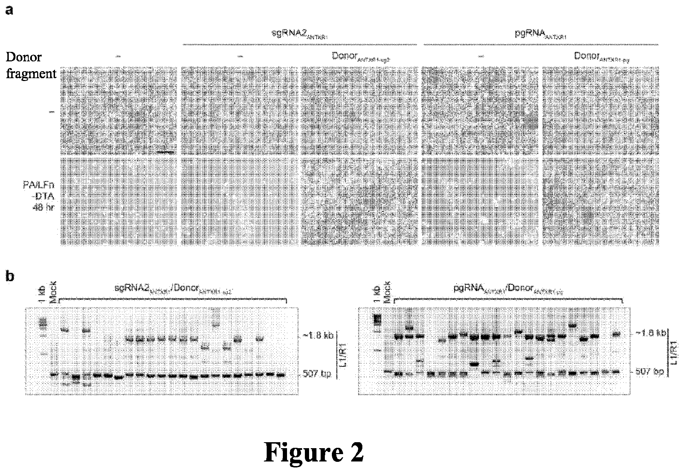

Figure 2 shows the experimental verification of enrichment of *ANTXR1* knockout cells in pooled population and single clones by donor-mediated puromycin resistance selection. (a) Images of different HeLa cell groups processed with or without PA/LFnDTA. Mixed cells were obtained by transfection using sgRNA or pgRNA with or without their corresponding linear donors. The scale is 200 µm. (b) PCR verification of the linear donor-integrated *ANTXR1* site of puromycin-resistant (puro+) single clones. The clones were obtained from HeLa cells transfected with sgRNA2_{ANTXR1}/Donor_{ANTXR1-sg2} (left) or pgRNA_{ANTXR1}/Donor_{ANTXR1-pg} (right).

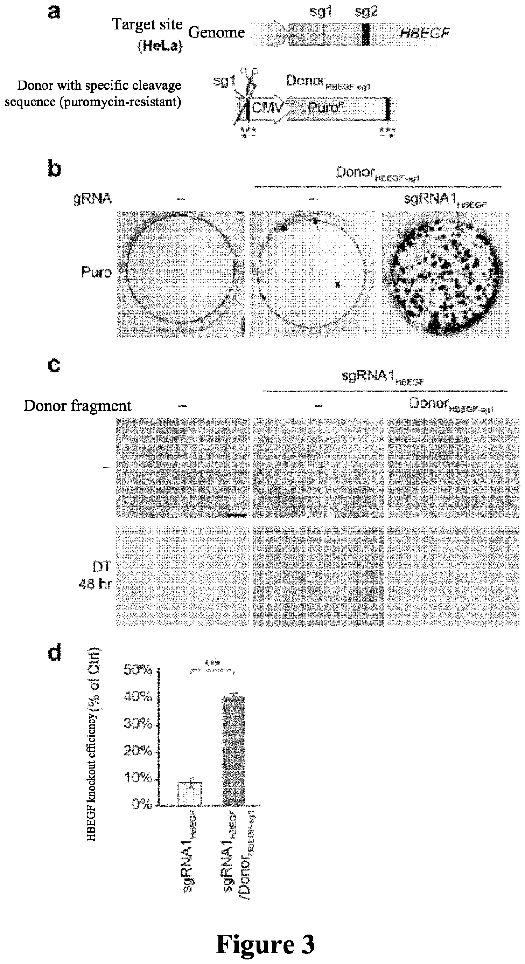

Figure 3 shows the donor design and experimental verification of enrichment of HEBGF destruction event in HeLa cells by puromycin selection. (a) Design of donor targeting *HBEGF* gene. (b) MTT staining of puromycin-resistant clones in cells transfected with linear donor Donor_{HBEGF-sg1}(with or without Cas9/sgRNA). (c) Images of different HeLa cell groups processed with or without DT (40 ng/ml). Mixed cells were obtained by transfection using sgRNA (sgRNA1_{HBEGF}) (with or without its corresponding linear donor (Donor_{HBEGF-sg1})). The scale is 200 µm. (d) *HBEGF* knockout rates of HeLa cells transfected with sgRNA (sgRNA1_{HBEGF}), plasmids expressing Cas9, and reporter plasmids containing puromycin-resistant genes (a light stripe), or with sgRNA, plasmids expressing Cas9, and linear donor (Donor_{HBEGF-sg1}) (a dark stripe). The *HBEGF* knockout rate is expressed as the percentage of cells resistant to DT. Cells were selected by puromycin (1 µg/ml) prior to DT resistance analysis. Error line indicates s.d. (n = 3), t-test, ***P < 0.001.

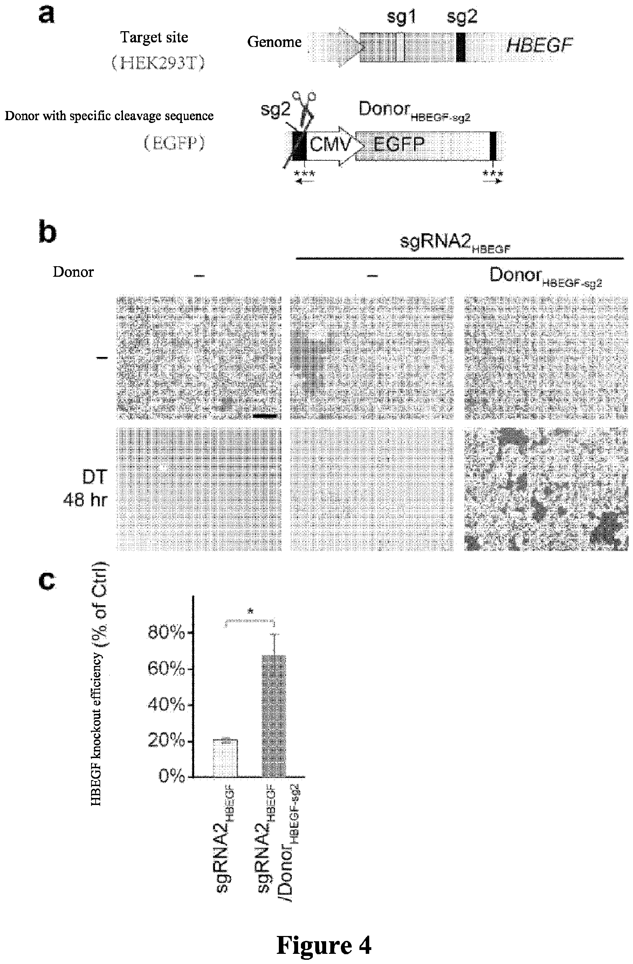

Figure 4 shows the donor design and experimental verification of enrichment of *HBEGF* destruction event in HEK293T cells via EGFP. (a) Design of donor targeting *HBEGF* gene. (b) Images of different HEK293T cell groups processed with or without DT (40 ng/ml). Mixed cells were obtained by transfection using sgRNA (sgRNA2_{HBEGF}) (with or without their corresponding linear donor (Donor_{HBEGF-sg2})). The scale is 200 µm. (c) *HBEGF* knockout rates of HEK293T cells transfected with sgRNA (sgRNA2_{HBEGF}) plasmids expressing mCherry, and plasmids expressing Cas9 (a light stripe), or with sgRNA, plasmids expressing Cas9, and a linear donor (Donor_{HBEGF-sg2}, EGFP) (a dark stripe). The *HBEGF* knockout rate is expressed as the percentage of cells resistant to DT. Cells were selected by FACS prior to DT resistance analysis. Error line indicates s.d. (n = 3), t-test, ***P < 0.05.

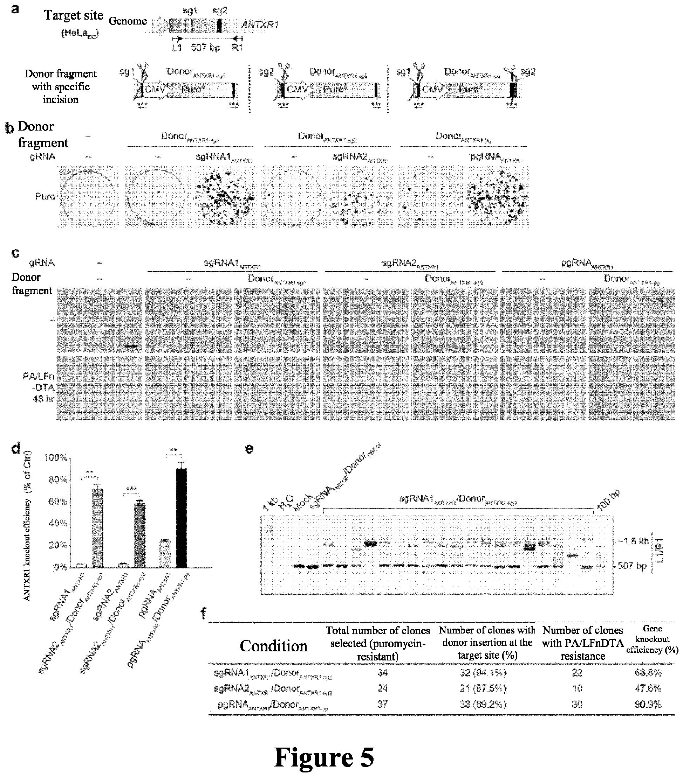

Figure 5 shows the donor design and experimental verification of enrichment of *ANTXR1* destruction event in HeLaoc cells by puromycin selection. (a) Design of donor targeting *ANTXR1*. The donor comprises an sgRNA cleavage site (Donor_{ANTXR1-sg1} or Donor_{ANTXR1-sg2}) at the 5'-end or two gRNAs (Donor_{ANTXR1-pg}) at both ends. (b) MTT staining of puromycin-resistant clones in cells transfected with a donor (with or without gRNA). (c) Images of different HeLaoc cell groups processed with or without PA/LFnDTA. Mixed cells were obtained by transfection using sgRNA or pgRNA with or without its corresponding linear donor. The scale is 200 µm. (d) *ANTXR1* knockout rates of HeLaoc cells transfected using sgRNAs with or without their corresponding donors. The *ANTXR1* knockout rate is expressed as the percentage of cells resistant to PA/LFnDTA. Cells were selected by puromycin (1 µg/ml) prior to PA/LFnDTA resistance analysis. Error line indicates s.d. (n = 3), t-test, ***P < 0.001. (e) PCR verification of the linear donor-integrated *ANTXR1* site of puromycin-resistant single clones. (f) Summary of *ANTXR1* knockout cells enriched via different gRNAs and their donors.

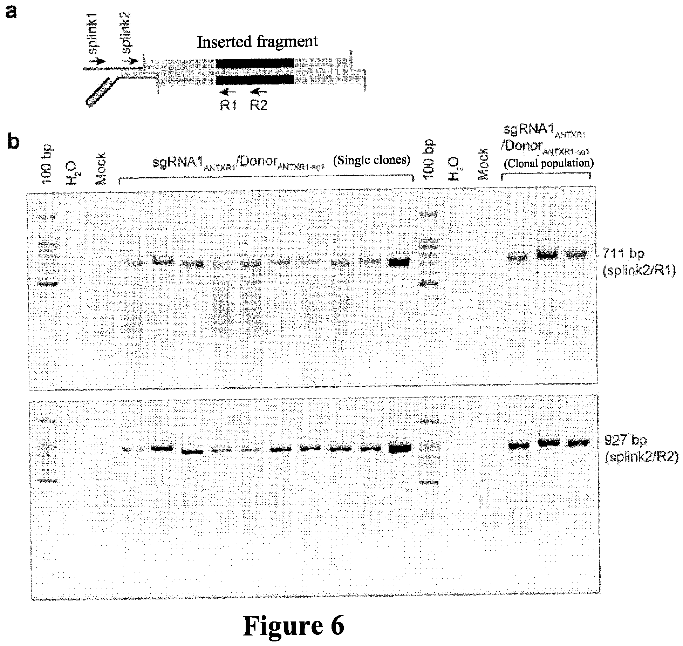

Figure 6 shows the off-target evaluation of donor insertion in HeLaoc cells by Splinkerette PCR (spPCR) analysis. (a) adaptor and primer design for spPCR analysis. Splink1 and Splink2 primers match the adaptor sequence, and primers R1 and R2 match the linear donor sequence. (b) spPCR reaction results.

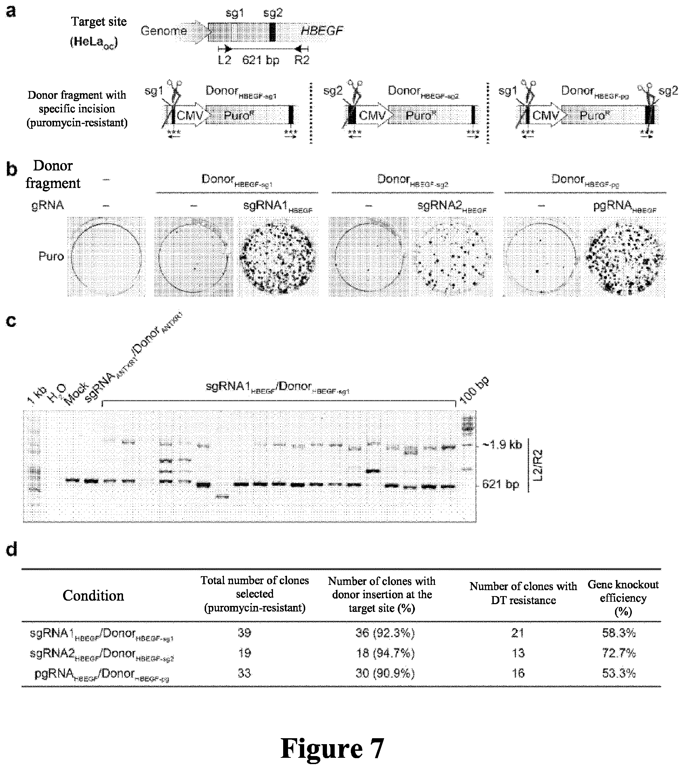

Figure 7 shows the donor design and experimental verification of enrichment of *HBEGF* destruction event in HeLaoc cells by puromycin selection. (a) Design of donor targeting *HBEGF.* The donor comprises an sgRNA cleavage site (Donor_{HBEGF-sg1} or D0nor_{HBEGF-sg2}) at the 5'-end or two gRNAs (Donor_{HBEGF-pg}) at both ends. (b) MTT staining of puromycin-resistant clones of cells transfected with a donor (with or without sgRNA/pgRNA). (c) PCR verification of the linear donor-integrated *HBEGF* site of puromycin-resistant single clones. (d) Summary of *HBEGF* knockout cells enriched via different gRNAs and their donors.

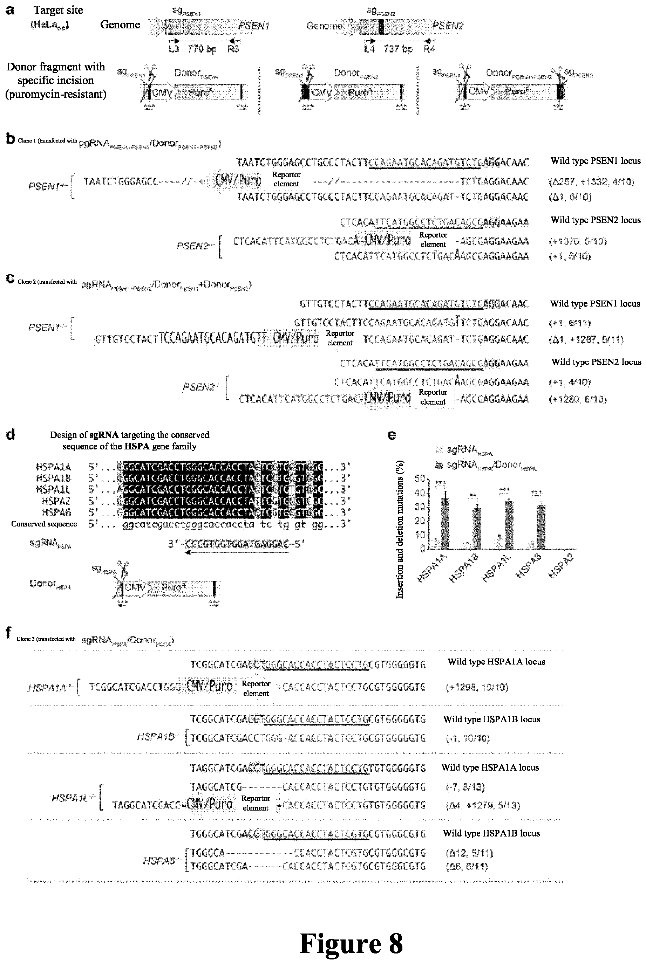

Figure 8 shows the donor design and experimental verification of generation of two or more gene knockouts in one step in HeLaoc cells. (a) Schematic diagram of knockin of linear donor at sgRNA- or pgRNA-targeting site in *PSEN1* and *PSEN2* genes based on NHEJ. (b-c) Sequencing analysis of partial encoding sequences of *PSEN1* and *PSEN2* in genome, containing sgRNA encoding region (underlined) and mutant alleles. Clone 1 (b) was derived from HeLaoc cells transfected with pgRNA_{PSEN1+PSEN2}/Donor_{PSEN1+PSEN2}. Clone 2 (c) was derived from HeLa_{OC} cells transfected with pgRNA_{PSEN1+PSEN2}/Donor_{PSEN1}+Donor_{PSEN2}. The nucleotides in the shaded region represent the PAM sequence that guides Cas9 to perform DNA recognition and cleavage. Dashed lines indicate deletions, high letters indicate nucleotide insertions, and light gray arrows in the background indicate the direction of the CMV promoter in the donor. (d) Multiple sequence alignment analysis of the *HSPA* gene family showing its consensus sequence and sgRNA targeting the consensus sequence of five *HSPA* family genes; and design of general linear donor (Donor_{HSPA}) for enrichment of cells containing multi-gene mutations. The dark shaded nucleotides represent the consensus sequence of all five *HSPA* genes. Dark gray shaded nucleotides represent the consensus sequence of three or four *HSPA* genes, while light gray shaded nucleotides represent non-consensus nucleotides. (e) indels triggered by sgRNA_{HSPA} in five target genes after puromycin selection in the absence and presence of Donor_{HSPA}. Error line indicates s.d. (n = 3), *t*-test, ***P*< 0.01, ****P* < 0.001. (f) Partial encoding sequences of *HSPA1A, HSPA1B, HSPA1L* and *HSPA6* genes in the genome of HeLa clone 3 containing sgRNA-targeting region (underlined). Clone 3 was derived from HeLaoc cells transfected with sgRNA_{HSPA}/Donor_{HSPA}. The shaded nucleotides represent the PAM sequence, and the dashed lines represent deletions. Light gray arrows in the background indicate the direction of the CMV promoter in the donor.

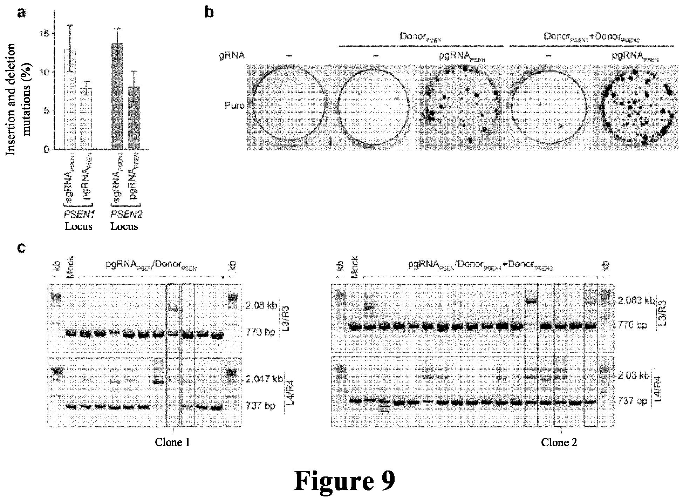

Figure 9 shows the efficiency evaluation and single clone recognition of *PSEN1* and *PSEN2* sgRNAs in HeLaoc cells. (a) Evaluation for efficiency of indels caused by sgRNA_{PSEN1}, SgRNA_{PSEN2} and pgRNA_{PSEN} at *PSEN1* and *PSEN2* sites through T7E1 analysis. Error line indicates s.d. (n = 3). (b) MTT staining of puromycin-resistant clones of cells transfected with a donor (with or without pgRNA_{PSEN}). (c) PCR results of the two linear donor-integrated sites, *PSEN1* (L3/R3) and *PSEN2* (L4/R4) of puromycin-resistant single clones.

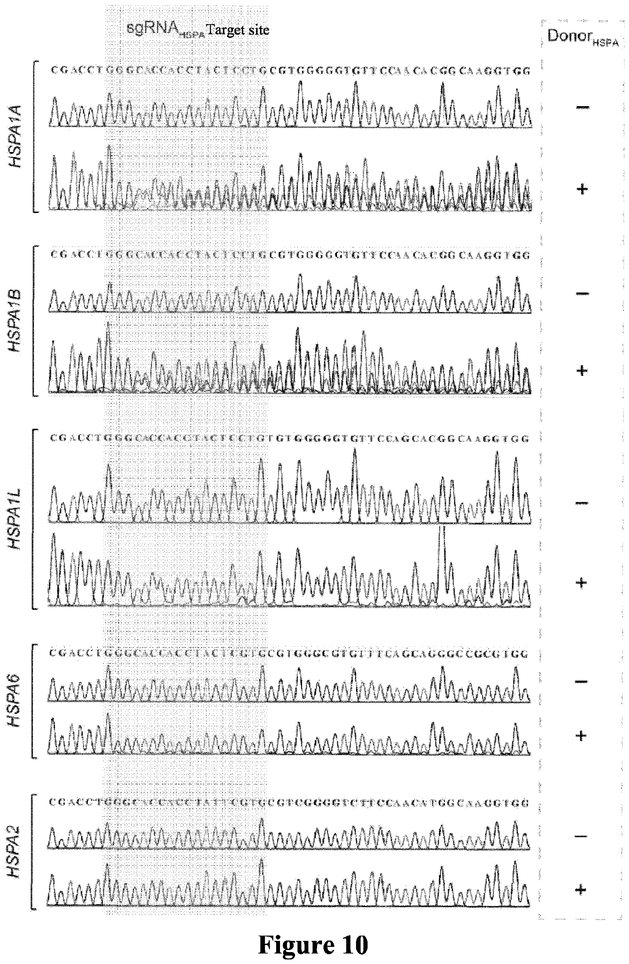

Figure 10 shows the sequencing maps of target regions of the *HSPA* family genes in mixed cells transfected with or without donor. The sgRNA-targeting sites are shaded, and target regions containing donor insertions are not included in these sequencing analyses.

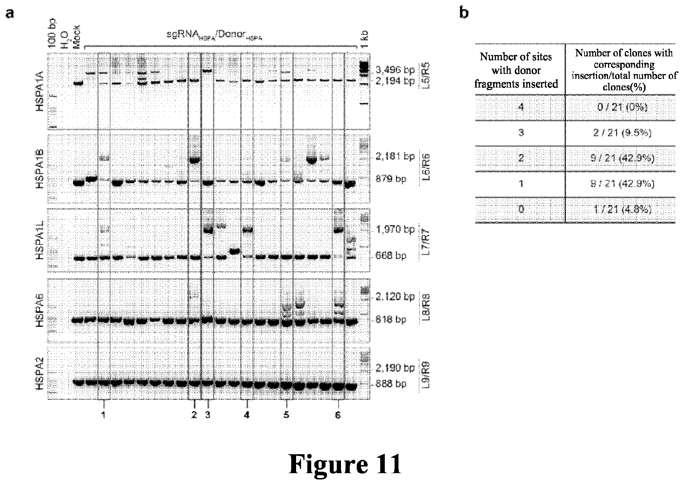

Figure 11 shows identification of single clones with inserted donor at target sites of five *HSPA* family genes, *HSPA1A, HSPA1B*, *HSPA1L*, *HSPA6* and *HSPA2.* (a) PCR verification results of linear donor integration of puromycin-resistant single clones at all five gene loci. (b) Summary of donor insertion results at four gene loci, *HSPA1A, HSPA1B, HSPA1L* and *HSPA6.*

### Detailed Description of Embodiments

The present invention provides a novel donor construct and a gene knockout method. The method uses a linear donor DNA to improve the production efficiency of gene knockout by a sequence-specific nuclease. The linear donor DNA of the present invention comprises at least one target site that can be cleaved by a sequence-specific nuclease. The target site contained in the linear donor DNA is designed according to the target site in the cell genome, so that a sequence-specific nuclease that can cleave the target site in the cell genome can also cleave the target site contained in the linear donor DNA. After a sequence-specific nuclease and a donor construct are introduced into a cell, the sequence-specific nuclease simultaneously cleaves at least one target site contained in the linear donor DNA when double-strand breaks (DSBs) are generated at a specific target site in the cell. This thus allows the linear donor DNA to be inserted into the cleaved target site in the cell genome by Non-Homologous end joining (NHEJ) pathway with a higher efficiency. Subsequent selection of cells through this marker can effectively enrich cells that are cleaved at the specific target site of the genome to produce gene knockout, thus greatly improving the production efficiency of gene knockout by the sequence-specific nuclease.

It has been reported that if one of the homologous alleles is modified, the mutation frequency of the target alleles is usually higher ^{[25,26]}. Thus, while not wishing to be bound by theory, the inventors speculate that if a donor can be inserted at a specific site in one of the target alleles and clones that express the donor's marker gene are selected, it might be possible to enrich those rare events where all the alleles are modified.

In the present invention, "gene knockout" is to realize the loss of gene functions through gene editing. The gene knockout effect that is usually pursued is simultaneous knockout of two alleles, at which time the corresponding protein loses its functions and a gene knockout cell line is obtained. If only one allele is knocked out, the protein can also play its partial role, i.e., the protein functions are only down-regulated. Cells with both alleles being knocked out can be enriched effectively by using the linear donor DNA of the present invention and the method of the present invention.

The donor construct of the present invention is a double-stranded DNA. The donor construct of the present invention may itself be a linear donor DNA. Alternatively, the donor construct of the present invention may be a circular DNA molecule comprising a linear donor DNA that, when introduced into a cell, is cleaved in the cell to produce the linear donor DNA. Methods of cleaving circular donor constructs in cells to produce linear donor DNAs are well known in the art. For instance, the circular construct can further comprise a cleavage site for another sequence-specific nuclease upstream of the 5'-end and downstream of the 3'-end of the linear donor DNA.

The method of the present invention may further comprise introducing another sequence-specific nuclease to the cell. The sequence-specific nuclease cleaves a sequence upstream of the 5'-end and downstream of the 3'-end of the linear donor DNA in the circular construct in the cell, thereby producing the linear donor DNA.

In the linear donor DNA of the present invention, "reverse termination codon" means that the codon is oriented in the opposite direction to the open reading frame of the expression cassette. "Forward termination codon" means that the codon is oriented in the same direction as the open reading frame of the expression cassette. The role of termination codons is that, regardless of whether the linear donor is inserted into the genome forward or backward, two triplet termination codons can terminate endogenous and exogenous gene expression.

The "protection sequence" in the linear donor DNA of the present invention can be any sequence, and the preferred protection sequence is different from the target sequence in the same linear donor DNA. The protection sequence can be 5-30 bp, and most preferably 20 bp, in length. The role of the protection sequence is to protect the target sequence in the linear donor DNA from being cleaved by an enzyme (e.g., an exonuclease) in the cell.

As used herein, a "marker gene" refers to any marker gene whose expression can be selected or enriched, i.e., when the marker gene is expressed in a cell, cells expressing the marker gene can be selected and enriched in a certain manner. Marker genes useful in the present invention include, but are not limited to, fluorescent protein genes that can be sorted by FACS after expression, or resistance genes that can be screened by antibiotics, or protein genes that can be recognized by corresponding antibodies after expression and screened by immunostaining or magnetic bead adsorption. Resistance genes useful in the present invention include, but are not limited to, resistance genes targeting Blasticidin, Geneticin (G-418), Hygromycin B, Mycophenolic Acid, Puromycin, Zeocin or Neomycin. Fluorescent protein genes useful in the present invention include, but are not limited to, genes of Cyan Fluorescent Protein, Green Fluorescent Protein, Yellow Fluorescent Protein, Orange Fluorescent Protein, Red Fluorescent Protein, Far-Red Fluorescent Protein, or Switchable Fluorescent Proteins.

Examples of sequence-specific nucleases include zinc finger nucleases (ZFNs). Zinc finger nucleases are non-naturally occurring and artificially modified endonucleases, which are composed of a zinc finger protein domain and a non-specific endonuclease domain. The zinc finger protein domain consists of a series of zinc finger proteins Cys2-His2 in series. Each zinc finger protein recognizes and binds to a specific triplet base on the DNA strand in the 3' to 5' direction and a base in the 5' to 3' direction. Multiple zinc finger proteins can be connected in series to form a zinc finger protein group, which can recognize a stretch of specific base sequence with strong specificity. The non-specific endonuclease linked to the zinc finger protein group is derived from the DNA cleavage domain consisting of 96 amino acid residues at the carboxyl end of FokI. Each FokI monomer is linked to a zinc finger protein group to form a ZFN that recognizes specific sites. When two recognition sites are at an appropriate distance (6-8 bp), two monomeric ZFNs interact with each other to produce an enzyme digestion function, so as to achieve the purpose of site-specific DNA cleavage. 8-10 zinc finger domains are designed for the target sequence. By attaching these zinc domains to DNA nucleases, Double-strand breaks (DSBs) of the target sequence can be produced, and DSBs repair mechanisms can be induced to conduct directional transformation of specific sites in the genome.

Another example of sequence-specific nucleases includes transcription activator-like effector nucleases (TALENs). Transcription activator-like effector nucleases are mainly composed of a Fok I endonuclease domain and a DNA binding domain of TALE protein. The TALE protein contains multiple repeating peptide segments of 33-35 amino acids, and each peptide segment recognizes one base. Like ZFNs, TALENs can also cleave DNA target sequences to form DSBs, thereby activating DNA damage repair mechanisms and performing site-specific transformation of the genome.

Another example of the sequence-specific nuclease system useful in the present invention includes Cas9/CRISPR (Clustered Regularly Interspaced Short Palindromic Repeats) systems. The Cas9/CRISPR systems utilize RNA-directed DNA binding for sequence-specific cleavage of target DNAs, and crRNA (CRISPR-derived RNA) binds to tracrRNA (trans-activating RNA) by base pairing to form a tracrRNA/crRNA complex. This complex directs the nuclease Cas9 protein to cleave the double-stranded DNA at a specific position in the target sequence that is paired with the crRNA. The target sequence paired with the crRNA is usually a sequence of about 20 nucleotides located upstream of the genomic PAM (protospacer adjacent motif) site (NNG).

The Cas9 protein cleaves the target site by means of a guide RNA. The term "guide RNA" is also known as gRNA. A gRNA typically comprises a nucleotide complementary to the target sequence on the crRNA and an RNA Scaffold formed by base pairing of the crRNA and the tracrRNA, and is capable of recognizing the target sequence paired with the crRNA. The gRNA can form a complex with the Cas9 protein and bring the Cas9 protein to the target sequence and cleave the target site therein.

The gRNA is commonly used in the form of an sgRNA (single guide RNA). sgRNA, also known as "single-stranded guide RNA", is an RNA strand formed by fusing the crRNA with the trancrRNA.

Another example of the sequence-specific nuclease system useful in the present invention includes NgAgo nucleases and their gDNAs. An NgAgo nuclease can bind to a single-stranded guide DNA (gDNA) phosphorylated at the 5'-end to cleave the target sequence complementary to this gDNA, thus producing DNA double-strand breaks.

The linear donor DNA of the present invention may have a target sequence only at one end, or may have target sequences at both ends, respectively. The target sequences at both ends of the linear donor DNA can be different. When gene knockout is required to be produced by cleavage at two different target sites in the cell genome, two linear donor DNAs can be provided, and each linear donor DNA comprises a corresponding target sequence, respectively; or alternatively, a linear donor DNA can also be provided, each end of which comprises a corresponding target sequence. When gene knockout is required to be produced by cleavage at a plurality of different target sites in the cell genome, an appropriate number of linear donor DNAs can be provided, and one or both ends of each linear donor DNA comprises one of a plurality of different corresponding target sequences, respectively. For instance, linear donor DNAs can be provided in the same amount as the number of the target sites, and each linear donor DNA comprises a corresponding target sequence, respectively. Alternatively, linear donor DNAs may be provided in an amount less than the number of the target sites, wherein both ends of all or part of the linear donor DNAs comprise one of a plurality of different corresponding target sequences, respectively, and each of the other linear donor DNAs comprises one of the other corresponding target sequences, respectively.

The sequence-specific nuclease can be introduced into a cell in the form of a protein or its encoding nucleic acid sequence (e.g., mRNA or cDNA). A nucleic acid encoding a sequence-specific nuclease can be introduced into a cell by inclusion in a plasmid or viral vector, e.g., introduced into a cell by transfection. A nucleic acid encoding a sequence-specific nuclease can also be delivered directly into a cell by electroporation, liposome, microinjection, and the like.

The donor construct can be delivered by any method suitable for introducing a nucleic acid into a cell, e.g., introduced into a cell by transfection.

In the case of producing gene knockout using the Cas9/CRISPR systems and NgAgo nucleases, an sgRNA or gDNA is also introduced into a cell. The sgRNA or gDNA can be delivered by any method suitable for introducing RNA or DNA into a cell. The sgRNA can be introduced into a cell in the form of an isolated RNA. Isolated sgRNAs can be prepared by *in vitro* transcription using any *in vitro* transcription system known in the art. The sgRNA can also be introduced into a cell by a vector comprising a sequence encoding the sgRNA and a promoter. The vector may be a viral vector or a plasmid. The means by which somewhat is introduced into a cell can be transfection. Two or more sgRNAs respectively targeting different target sites can be introduced into a cell to direct cleavage by Cas9 at two or more different target sites in the cell genome to produce gene knockout. The two or more sgRNAs may be contained in different vectors, or may be contained in the same vector, such as a vector comprising a pair of gRNAs (paired gRNA), or a vector comprising more sgRNAs.

In the method of the present invention, when two or more sgRNAs respectively targeting different target sites are introduced into a cell, a linear donor DNA comprising target sequences recognized by these sgRNAs is simultaneously introduced. Since the linear donor DNA may comprise a target sequence only at the 5'-end or 3'-end, or may also comprise target sequences at both ends, respectively, the number of sgRNAs and the number of linear donor DNAs can be different. It can be one sgRNA corresponding to one linear donor DNA, or two sgRNAs corresponding to two linear donor DNAs.

Preferably, in the present invention, in the case of producing gene knockout using the Cas9/CRISPR systems, Cas9, an sgRNA and a linear donor DNA can be simultaneously introduced into a cell; or alternatively, for instance, Cas9 can be first introduced into a cell, and then an sgRNA and a linear donor DNA are introduced into the cell. In some embodiments, cells are co-transfected with a vector comprising Cas9, a vector comprising an sgRNA, and a linear donor DNA. In still some embodiments, Cas9 and an sgRNA are assembled *in vitro* into a protein-RNA complex, which is used to co-transfect cells with a linear donor DNA. In still some embodiments, Cas9 and an sgRNA are stably expressed into cells by lentiviruses, and the cells are transfected with a linear donor DNA. In other embodiments, Cas9 is first stably expressed in cells, and the cells are then co-transfected with a vector comprising an sgRNA and a linear donor DNA.

In the system or kit provided by the present invention for gene knockout, a sequence-specific nuclease may be in the form of a protein or its encoding nucleic acid sequence (e.g., mRNA or cDNA), such as in the form of a plasmid comprising a nucleic acid encoding a sequence-specific nuclease or a viral vector. In the case of using the Cas9/CRISPR systems, an sgRNA may be in the form of an isolated RNA or a vector comprising a sequence encoding the sgRNA and a promoter, such as a viral vector or a plasmid vector.

The cell described herein can be any eukaryotic cell, such as an isolated animal cell, e.g., a totipotent cell, a pluripotent cell, an adult stem cell, a fertilized egg, a somatic cell, or the like. In some embodiments, the cell is a vertebrate cell. In some embodiments, the cell is a mammalian cell. In some embodiments, the cell is a human cell. In some embodiments, the cell is a cell derived from cow, goat, sheep, cat, dog, horse, rodent, fish, and primate. In some embodiments, rodents include mice, rats, and rabbits.

The method of the present invention can be used to perform targeted gene knockout in a single gene or multiple genes in a cell, such as targeting two, three, four, five or more gene knockouts. Targeted gene knockout for multiple genes can be performed simultaneously or successively. For instance, a sequence-specific nuclease or a sequence-specific nuclease system for two or more target genes can be introduced into a cell and then cells are subjected to enrichment screening. Alternatively, a sequence-specific nuclease or a sequence-specific nuclease system for one or more target genes can be first introduced into a cell and cells are subjected to enrichment screening, and then a sequence-specific nuclease or a sequence-specific nuclease system for other target genes can be introduced into the cell and cells are subjected to enrichment screening. Different marker/marker genes can be used for different target genes. For instance, in the case of producing gene knockout using the Cas9/CRISPR systems, two or more sgRNAs respectively targeting different target sites can be introduced into a cell, and a linear donor DNA comprising target sequences recognized by these sgRNAs is simultaneously introduced, as previously mentioned. When these different target sites are located on different genes, gene knockout of multiple genes can be achieved. The sgRNA and target sequences in a linear donor DNA can also be designed by using the consensus sequence of two or more genes in the cell genome. At this point, an sgRNA recognizing a single specific target site in the cell genome can be introduced into a cell, and a linear donor DNA comprising a target sequence recognized by the sgRNA is simultaneously introduced, wherein the target sequence recognized by the sgRNA is the consensus sequence of two or more genes in the cell genome, provided that the consensus sequence has no more than one base difference from the sequence at positions corresponding to the consensus sequence in any of the two or more genes. Two-base difference may disrupt recognition by an sgRNA, as demonstrated in Example 7.

The target gene to which gene editing by the linear donor DNA of the present invention is targeted is not particularly limited, as long as it can produce double-strand breaks by the Cas9/CRISPR systems. The target gene may be an exon, an intron or a regulatory sequence, or any combination thereof.

The term "including" or "comprising", as used in the present invention, indicates "including, but not limited to", "consisting essentially of" or "consisting of".

The present invention is further illustrated in conjunction with the following examples and the accompanying drawings, which are used for illustration purposes only and are not intended to limit the scope of the present invention. If not specially stated, the examples are all conducted in accordance with normal experimental conditions, such as those described in Sambrook J & Russell DW, Molecular cloning: a laboratory manual, 2001, or in accordance with the instructions provided by the manufacturers.

### Example 1 Enrichment of knockout events on ANTXR1 gene in HeLa cells using linear donor DNA

### 1. Design of sgRNAs

Two sgRNAs targeting the first exon of *ANTXR1* gene in HeLa cells were designed, and their efficiency in producing deletions or insertion mutations (Indels) at target sites was verified by T7E1 assay. The verification results are shown in Table 1. The target sequence to which sgRNA1_{ANTXR1} is targeted is referred to as sgl in this example, and the target sequence to which sgRNA2_{ANTXR1} is targeted is referred to as sg2 in this example.

**Table 1. sgRNAs Targeting the First Exon of ANTXR1 Gene in HeLa Cells**

| sgRNA | Target sequence (PAM) (5' to 3') | Mean Indels ± s.d. (%, n = 3) |
|---|---|---|
| sgRNA1_{ANTXR1} | AGCGGAGAGCCCTCGGCAT(CGG) | 19.97 ± 1.84 |
| sgRNA2_{ANTXR1} | TGCTCATCTGCGCCGGGCAA(GGG) | 16.83 ± 2.05 |

### 2. Construction of linear donor DNAs

A total of two linear donor DNAs (Donor_{ANTXR1-sg2} and Donor_{ANTXR1-pg}) were constructed, the structures of which are shown in Figure 1a.

Donor_{ANTXR1-sg2} includes, from 5'-end to 3'- end: 20 bp protection sequence, sg2, reverse termination codon, CMV promoter-driven puromycin-resistant gene, forward termination codon, and 20 bp protection sequence, respectively.

Donor_{ANTXR1-pg} includes, from 5'-end to 3'-end: 20 bp protection sequence, sg1, reverse termination codon, CMV promoter-driven puromycin-resistant gene, forward termination codon, sg2, and 20 bp protection sequence, respectively.

Linear donor DNA (Donor_{no cut}) as a control includes, from 5'-end to 3'-end: 20 bp protection sequence, 20 bp random sequence, reverse termination codon, CMV promoter-driven puromycin-resistant gene, forward termination codon, and 20 bp protection sequence, respectively. The random sequence is different from sgl or sg2.

### 3. Transfection

HeLa cells were co-transfected with a plasmid expressing Cas9, sgRNA2_{ANTXR1} or pgRNA_{ANTXR1}, and their corresponding donors. As a control, HeLa cells were transfected with linear donor DNAs (Donor_{ANTXR1-sg2}, Donor_{ANTXR1-pg}, and Donor_{no cut}) alone. To the cells puromycin was added for resistance screening. pooled population and single clones were obtained and stained with MTT (3-(4,5-dimethylthiazol-2-yl)-2,5-diphenyltetrazole bromide). The results are shown in Figure 1b.

A number of puromycin-resistant (puro+) cell clones were obtained from samples receiving sgRNA2_{ANTXR1} and its corresponding donor Donor_{ANTXR1-sg2}, and from samples receiving pgRNA_{ANTXR1} and its corresponding donor Donor_{ANTXR1-pg}. Only a few puromycin-resistant clones were produced by transfection with donors alone, probably because integration of linear donors into the chromosome was rare and random. In addition, co-transfection using the control donor Donor_{no cut} with the plasmid expressing Cas9 and sgRNA2_{ANTXR1} also failed to produce a significant number of puro+ clones (see the rightmost panel in Figure 1b), indicating that sgRNA-mediated cleavage by Cas9 in a donor is important for efficient donor integration. Integration of pgRNA_{ANTXR1}-mediated double-cleaved Donor_{ANTXR1-pg} was more efficient than sgRNA2_{ANTXR1} plus Donor_{ANTXR1-sg2}; however, no matter which linear donor DNA was used, sufficient puro+ clones were produced for subsequent mutant identification.

### 4. Verification of gene knockout efficiency

In addition, HeLa cells were co-transfected with a plasmid expressing Cas9 and sgRNA2_{ANTXR1} or pgRNA_{ANTXR1}, with or without their corresponding donors. Pooled population and single clones were obtained by screening with puromycin (1 µg/ml), wherein HeLa cells were co-transfected with a plasmid expressing the puromycin-resistant gene when no corresponding donor were added. As the *ANTXR1* gene knockout in HeLa cells results in resistance of the cells to chimeric anthrax toxin (PA/LFnDTA) ^{[17]}, pooled population and single clones obtained by puromycin screening were treated with PA/LFnDTA (PA: 150 ng/ml; and LFnDTA: 100 ng/ml) to compare the effect of linear donor DNAs on the *ANTXR1* knockout efficiency. Images of different cells treated with PA/LFnDTA are shown in Figure 2a. The *ANTXR1* knockout efficiency was determined by calculating the percentage of cells with this toxin resistance in the puro+ pooled population, as shown in Figure 1c. Compared to sgRNA2_{ANTXR1} alone or pgRNA_{ANTXR1} alone, the use of linear donor DNAs improved the gene knockout efficiency by 6-8 times. PCR verification was performed on the linear donor-integrated ANTRX1 site of puro+ single clones. The L1/R1 primer sequences used in PCR amplification are shown in Table 2. The results are shown in Figure 2b, and it can be seen that most of the clones isolated from the puro+ cell mixture contain donor inserts at the sgRNA-targeting sites (see also Figure 1d). It can also be seen from Figure 1d that nearly 90% of the cells carrying the donor fragment are true knockout clones.

**Table 2. Primers for Amplifying the Linear Donor-Integrated ANTRX1 Site in HeLa Cells**

| Primer pair | Sequence |
|---|---|
| L1/R1 | 5'-AAGCGGAGGACAGGATTGGG-3'/5'-CCTCTGTGGCCCTGGAGATG-3' |

### Example 2 Enrichment of knockout events on HBEGF gene in HeLa cells using linear donor DNA

Since a donor with a single- or double-cleavage site is capable of greatly increasing the selection of cells with modifications at the target site, for convenience, in this example, only a single-cleavage donor was adopted.

### 1. Design of sgRNAs

Two sgRNAs targeting the *HBEGF* gene in HeLa cells were designed, and their efficiency in producing Indels at target sites was verified by T7E1 assay. The verification results are shown in Table 3. The target sequence to which sgRNA1_{HBEGF} is targeted is referred to as sgl in this example, and the target sequence to which sgRNA2_{HBEGF} is targeted is referred to as sg2 in this example.

**Table 3. sgRNAs Targeting HBEGF Gene in HeLa Cells**

| sgRNA | Target sequence (PAM) (5' to 3') | Mean Indels ± s.d. (%, n = 3) |
|---|---|---|
| sgRNA1_{HBEGF} | GACTGGCGAGAGCCTGGAG(CGG) | 32.03 ± 7.13 |
| sgRNA2_{HBEGF} | CGGACCAGCTGCTACCCCT(AGG) | 14.27 ± 0.90 |

### 2. Construction of linear donor DNA

A linear donor DNA (Donor_{HBEGF-sg1}) was constructed, the structure of which is shown in Figure 3a.

Donor_{HBEGF-sg1} includes, from 5'-end to 3'- end: 20 bp protection sequence, sg1, reverse termination codon, CMV promoter-driven puromycin-resistant gene, forward termination codon, and 20 bp protection sequence, respectively.

### 3. Transfection

HeLa cells were co-transfected with a plasmid expressing Cas9, sgRNA1_{HBEGF}, and its corresponding donor Donor_{HBEGF-sg1}. As a control, HeLa cells were transfected with donor Donor_{HBEGF-sg1} alone. To the cells puromycin was added for resistance screening. Pooled population and single clones were obtained and stained with MTT. The results are shown in Figure 3b.

Similar to the results of Example 1, only the donor plus sgRNA obtained a large number of puro+ clones. This result again demonstrates that donor insertion depends on specific sgRNA/Cas9-mediated DSBs.

### 4. Verification of gene knockout efficiency

In addition, HeLa cells were co-transfected with a plasmid expressing Cas9 and sgRNA1_{HBEGF}, with or without its corresponding donor Donor_{HBEGF-sg1}. Pooled population and single clones were obtained by screening with puromycin (1 µg/ml), wherein HeLa cells were co-transfected with a plasmid expressing the puromycin-resistant gene when no corresponding donor were added. As the *HBEGF* gene encodes a diphtheria toxin (DT) receptor, and knocking it out in HeLa cells would result in resistance of the cells to DT ^{[17]}, pooled population and single clones obtained by puromycin screening were treated with DT (40 ng/ml) to compare the effect of linear donor DNAs on the *HBEGF* knockout efficiency. Images of different cells treated with DT are shown in Figure 3c. The *HBEGF* knockout efficiency was determined by calculating the percentage of cells with DT resistance in the puro+ pooled population, as shown in Figure 3d. As can be seen from Figure 3c and Figure 3d, the use of linear donor DNA greatly improves the *HBEGF* gene knockout efficiency compared to sgRNA1_{HBEGF} alone.

### Example 3 Enrichment of knockout events on HBEGF gene in HEK293T cells using linear donor DNA

### 1. Design of sgRNA and construction of linear donor DNA

sgRNA2_{HBEGF} targeting the *HBEGF* gene in HEK293T cells was designed and a linear donor DNA (Donor_{HBEGF-sg2}) was constructed. The donor includes, from 5'-end to 3'-end: 20 bp protection sequence, sg2, reverse termination codon, CMV promoter-driven *EGFP* gene, forward termination codon, and 20 bp protection sequence, respectively, as shown in Figure 4a.

### 2. Verification of gene knockout efficiency

HEK293T cells were co-transfected with a plasmid expressing Cas9 and sgRNA2_{HBEGF}, with or without its corresponding donor Donor_{HBEGF-sg2}. Cells were screened by FACS. The group with the donor added was screened for EGFP-positive cells by FACS, while the group without the donor added was screened for mCherry-positive cells by FACS. FACS-selected cells were treated with DT (40 ng/ml) to compare the effect of linear donor DNAs on the *HBEGF* knockout efficiency. Images of different cells treated with DT are shown in Figure 4b. The *HBEGF* knockout efficiency was determined by calculating the percentage of cells with DT resistance in the EGFP-positive cells, as shown in Figure 4c. The use of linear donor DNA greatly improves the *HBEGF* gene knockout efficiency compared to sgRNA2_{HBEGF} alone.

### Example 4 Enrichment of knockout events on ANTXR1 gene in HeLaoc cells using linear donor DNA

### 1. Establishment of HeLaoc cell line

The HeLaoc cell line was established according to the existing method ^{[17]}, and the cell line stably expresses Cas9.

### 2. Design of sgRNAs and construction of linear donor DNAs

Two sgRNAs (sgRNA1_{ANTXR1} and sgRNA2_{ANTXR1}) targeting the *ANTXR1* gene in HeLaoc cells were designed, and three linear donor DNAs (Donor_{ANTXR1-sg1}, Donor_{ANTXR1-sg2} and Donor_{ANTXR1-pg}) were constructed, as shown in Figure 5a. The target sequence to which sgRNA1_{ANTXR1} is targeted is referred to as sgl in this example, and the target sequence to which sgRNA2_{ANTXR1} is targeted is referred to as sg2 in this example.

Donor_{ANTXR1-sg1} includes, from 5'-end to 3'- end: 20 bp protection sequence, sg1, reverse termination codon, CMV promoter-driven puromycin-resistant gene, forward termination codon, and 20 bp protection sequence, respectively.

Donor_{ANTXR1-sg2} includes, from 5'-end to 3'- end: 20 bp protection sequence, sg2, reverse termination codon, CMV promoter-driven puromycin-resistant gene, forward termination codon, and 20 bp protection sequence, respectively.

Donor_{ANTXR1-pg} includes, from 5'-end to 3'-end: 20 bp protection sequence, sg1, reverse termination codon, CMV promoter-driven puromycin-resistant gene, forward termination codon, sg2, and 20 bp protection sequence, respectively.

### 3. Transfection

HeLaoc cells were co-transfected with a plasmid expressing Cas9, sgRNA1_{ANTXR1} or sgRNA2_{ANTXR1} or pgRNA_{ANTXR1}, and their corresponding donors. As a control, HeLaoc cells were transfected with linear donor DNAs (Donor_{ANTXR1-sg1}, Donor_{ANTXR1-sg2}, and Donor_{ANTXR1-pg}) alone. To the cells puromycin was added for resistance screening, and the cells were stained with MTT. The results are shown in Figure 5b.

Similar to the results in HeLa cells, only the donors plus sgRNAs obtained a large number of puro+ clones.

### 4. Verification of gene knockout efficiency

In addition, HeLaoc cells were co-transfected with a plasmid expressing Cas9 and sgRNA1_{ANTXR1} or sgRNA2_{ANTXR1} or pgRNA_{ANTXR1}, with or without their corresponding donors, and screened with puromycin (1 µg/ml). The screened cells were treated with PA/LFnDTA. Images of different cells treated with PA/LFnDTA are shown in Figure 5c. The *ANTXR1* knockout efficiency was determined by calculating the percentage of cells with this toxin resistance in the puro+ pooled population, as shown in Figure 5d. The use of linear donor DNAs greatly improves the *ANTXR1* gene knockout efficiency compared to sgRNAs alone.

For puro+ single clones, PCR verification was performed on the integration site of donor Donor_{ANTXR1-sg1} in the ANTXR1 gene. It was found that most of the puro+ clones contain donor inserts at the sgRNA-targeting sites (Figure 5e and Figure 5f), and most of the cells carrying donor fragments are true gene knockout clones (Figure 5f).

A PCR fragment of ∼500 bp (the length corresponding to the wild type *ANTXR1* gene) and a PCR fragment of ∼1.8 kb (the length corresponding to the wild type *ANTXR1* gene plus donor insert) were subjected to genome sequencing. The results are shown in Table 4.

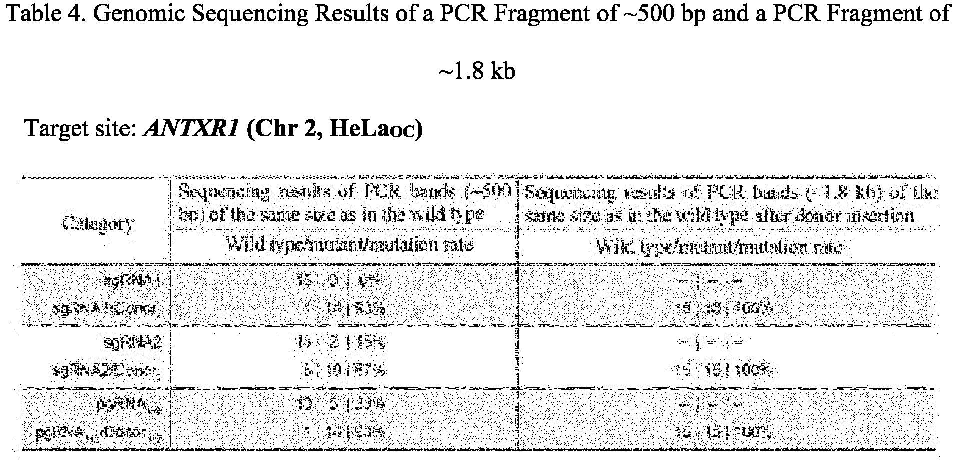

As can be seen from the PCR verification results (Figure 5e) and sequencing results (Table 4), most clones contain only one donor insert. However, in donor-positive clones, the vast majority of alleles are edited (or mutated) at the target site, but sgRNAs alone are inefficient in producing insertion or deletion mutations (indels) without using donor enrichment. This finding clearly demonstrates that donor insertion is closely related to the role of sgRNAs or pgRNAs.

### 5. Effect of donors on off-target effect of CRISPR/Cas systems

To examine whether the use of external donors would affect the off-target effect of the CRISPR/Cas systems, a whole genome integration site was found by Splinkerette PCR analysis [35-37]

In this example, the off-target insertions in single clones and mixed cell clones were verified by Splinkerette PCR analysis after puromycin selection. If the correct donor insertion is produced in the *ANTXR1* gene, amplification with primers Splink2/R1 and Splink2/R2 will result in 711- and 927-bp products, respectively (see Figure 6a).

For Splinkerette PCR analysis, we randomly selected 10 single clones with donor insertion and 4 puro+ mixed clones targeting *ANTXR1* in HeLaoc cells. Based on the Splinkerette PCR results (Figure 6b), similar to those clones that were not transfected with donors, there is no detectable off-target effect by single clones or pooled population enriched by donors.

### Example 5 Enrichment of knockout events on HBEGF gene in HeLaoc cells using linear donor DNA

### 1. Design of sgRNAs and construction of linear donor DNAs

Two sgRNAs (sgRNA1_{HBEGF} and sgRNA2_{HBEGF}) targeting the *HBEGF* gene in HeLaoc cells were designed, and three linear donor DNAs (Donor_{HBEGF-sg1}, Donor_{HBEGF-sg2} and Donor_{HBEGF-pg}) were constructed as shown in Figure 7a. The target sequence to which sgRNA1_{HBEGF} is targeted is referred to as sgl in this example, and the target sequence to which sgRNA2_{HBEGF} is targeted is referred to as sg2 in this example.

Donor_{HBEGF-sg1} includes, from 5'-end to 3'- end: 20 bp protection sequence, sg1, reverse termination codon, CMV promoter-driven puromycin-resistant gene, forward termination codon, and 20 bp protection sequence, respectively.

Donor_{HBEGF-sg2} includes, from 5'-end to 3'- end: 20 bp protection sequence, sg2, reverse termination codon, CMV promoter-driven puromycin-resistant gene, forward termination codon, and 20 bp protection sequence, respectively.

Donor_{HBEGF-pg} includes, from 5'-end to 3'-end: 20 bp protection sequence, sg1, reverse termination codon, CMV promoter-driven puromycin-resistant gene, forward termination codon, sg2, and 20 bp protection sequence, respectively.

### 3. Transfection

HeLaoc cells were co-transfected with a plasmid expressing Cas9, sgRNA1_{HBEGF} or sgRNA2_{HBEGF} or pgRNA_{HBEGF}, and their corresponding donors. As a control, HeLa_{OC} cells were transfected with linear donor DNAs (Donor_{HBEGF-sg1}, Donor_{HBEGF-sg2}, and Donor_{HBEGF-pg}) alone. To the cells puromycin was added for resistance screening. The results are shown in Figure 7b.

Similar to the results in HeLa cells, only the donors plus sgRNAs obtained a large number of puro+ clones.

### 4. Verification of gene knockout efficiency

For puro+ single clones, PCR verification was performed on the integration site of donor Donor_{HBEGF-sg1} in the *HBEGF* gene. The L2/R2 primer sequences used in PCR amplification are shown in Table 5. It was found that most of the puro+ clones contain donor inserts at the sgRNA-targeting sites (Figure 7c and Figure 7d), and most of the cells carrying donor fragments are true gene knockout clones (Figure 7d).

**Table 5. Primers for Amplifying the Linear Donor-Integrated HBEGF Site in HeLaoc Cells**

| Primer pair | Sequence |
|---|---|
| L2/R2 | 5'-GCCGCTTCGAAAGTGACTGG-3'/5'-GATCCCCCAGTGCCCATCAG-3' |

### Example 6 Double gene knockout in HeLaoc cells using linear donor DNA

### 1. Design of sgRNAs

Two target genes in HeLaoc cells were selected: *PSEN1* and *PSEN2.* Two sgRNAs respectively targeting these two target genes were designed, and their efficiency in producing Indels at target sites was verified by T7E1 assay. The results are shown in Table 6. The target sequence to which sgRNA_{PSEN1} is targeted is referred to as sg_{PSEN1} in this example, and the target sequence to which sgRNA_{PSEN2} is targeted is referred to as sg_{PSEN2} in this example.

**Table 6. sgRNAs Targeting PSEN1 and PSEN2 Genes in HeLaoc Cells**

| sgRNA | Target sequence (PAM) (5' to 3') | Mean Indels ± s.d. (%, n = 3) |
|---|---|---|
| sgRNA_{PSEN1} | CCAGAATGCACAGATGTCTG(AGG) | 13.03 ± 3.04 |
| sgRNA_{PSEN2} | TTCATGGCCTCTGACAGCG(AGG) | 13.67 ± 0.55 |

### 2. Construction of linear donor DNAs

Two types of donors were constructed. One type had two separate donors (Donor_{PSEN1}+Donor_{PSEN2}), and each donor had a target sequence for the corresponding sgRNA; and the other type of donor (Donor_{PSEN}) had two sgRNA-targeting sequences at both ends, respectively, as shown in Figure 8a. Donor_{PSEN1} or Donor_{PSEN2} had a cleavage site for sgRNA_{PSEN1} or sgRNA_{PSEN2} at its 5'-end. Donor_{PSEN1+PSEN2} had a cleavage site for sgRNA_{PSEN1} at the 5'-end and a cleavage site for sgRNA_{PSEN2} at the 3'-end. Among the donors:
Donor_{PSEN1} includes, from 5'-end to 3'- end: 20 bp protection sequence, sg_{PSEN1}, reverse termination codon, CMV promoter-driven puromycin-resistant gene, forward termination codon, and 20 bp protection sequence, respectively.

Donor_{PSEN2} includes, from 5'-end to 3'- end: 20 bp protection sequence, sg_{PSEN2}, reverse termination codon, CMV promoter-driven puromycin-resistant gene, forward termination codon, and 20 bp protection sequence, respectively.

Donor_{PSEN} includes, from 5'-end to 3'-end: 20 bp protection sequence, sg_{PSEN1}, reverse termination codon, CMV promoter-driven puromycin-resistant gene, forward termination codon, sg_{PSEN2}, and 20 bp protection sequence, respectively.

### 3. Verification of transfection and gene knockout efficiency

HeLaoc cells were co-transfected with a plasmid expressing Cas9, sgRNA_{PSEN1} or sgRNA_{PSEN2}; or alternatively, HeLaoc cells were co-transfected with a plasmid expressing Cas9 and pgRNA_{PSEN} to produce indels at specific sites of the PSEN1 and PSEN2 genes. T7E1 analysis was performed on the production efficiency of indels ^{[26]} (see Table 7 for the primers used). The results are shown in Figure 9a, and their co-transfection all show only ordinary activity.

HeLaoc cells were co-transfected with a plasmid expressing Cas9, pgRNA_{PSEN}, and Donor_{PSEN}; or alternatively, HeLaoc cells were co-transfected with a plasmid expressing Cas9, pgRNA_{PSEN}, and Donor_{PSEN1}+Donor_{PSEN2}. To the cells puromycin was added for resistance screening to obtain puro+ clones (see Figure 9b). Similar to the results of the previous examples, the donors plus pgRNAs obtained a large number of puro+ clones.

For each transfection result, puro+ single clones were subjected to PCR verification of the integration sites of donors Donor_{PSEN} and Donor_{PSEN1}+Donor_{PSEN2} in the *PSEN1* and *PSEN2* genes. The primers used in the PCR amplification are shown in Table 7, wherein L3/R3 was used to amplify the integration site in *PSEN1*, and L4/R4 was used to amplify the integration site in *PSEN2*. The PCR verification results are shown in Figure 9c. Clones containing donor insertions in both genes are indicated by boxes. Clone 1 and clone 2 were selected for further genome sequencing analysis, and both clones showed disruption of *PSEN1* and *PSEN2* (Figure 8b and Figure 8c).

**Table 7. Primers for Amplifying the Linear Donor-Integrated PSEN1 and PSEN2 Sites in HeLaoc Cells**

| Primer pair | Sequence |
|---|---|
| L3/R3 | 5'-TGGTGTCTCAGGCGGTTCTA-3'/5'-TGAACTATGAGGCGCTGCAC-3' |
| L4/R4 | 5'-TGACTTTCGTGGCTATGCGT-375'-CTAGCACCCAGGCATCCAAA-3' |

### Example 7 Multi-gene knockout in HeLaoc cells using linear donor DNA

### 1. Selection of target genes and design of sgRNA

The *HSPA* gene family in HeLaoc cells was selected, which includes five homologous genes, *HSAPA1A, HSPA1B, HSBA1L, HSPA6* and *HSPA2.* sgRNA_{HSPA} simultaneously targeting *HSAPA1A, HSPA1B* and *HSBA1L* was designed. The target sequence of the sgRNA has a mismatch with the corresponding sequence in *HSPA6* and two mismatches with *HSPA2,* as shown in Figure 8d.

### 2. Construction of linear donor DNA

A linear donor Donor_{HSPA} was constructed, including, from 5'-end to 3'- end: 20 bp protection sequence, sg_{HSPA}, reverse termination codon, CMV promoter-driven puromycin-resistant gene, forward termination codon, and 20 bp protection sequence, respectively (Figure 8d).

### 3. Verification of transfection and gene knockout efficiency

HeLaoc cells were co-transfected with a plasmid expressing Cas9 and sgRNA_{HSPA}, with or without its corresponding donor Donor_{HSPA}, indels were triggered, and resistance screening was performed by puromycin. The group without the donor added was co-transfected with a plasmid expressing a puromycin-resistant gene. The indels efficiency of all five genes was evaluated by T7E1 assay (see Table 8 for the primers used). The results are shown in Figure 8e. Compared with sgRNA_{HSPA} alone, the use of donor *HSPA* increased the mutation rate at the *HSPA1A* site by approximately 5.5 times, increased the mutation rate at the *HSPA1B* site by approximately 6.1 times, increased the mutation rate at the *HSPA1L* site by approximately 3.4 times, and increased the mutation rate at the *HSPA6* site by approximately 6.6 times. Interestingly, no indels was detected in the *HSPA2* gene, whether or not the donor was used, indicating that the two mismatches completely disrupt recognition by sgRNA_{HSPA}. Furthermore, and more importantly, selection using a donor did not increase the risk of off-target effects.

The target regions of *HSPA* family genes in mixed cells obtained by co-transfection with and without the donor were sequenced. The results are shown in Figure 10. The results showed that the cell mixed sequencing results are consistent with the results of T7E1 assay, regardless of whether there is donor transfection at the *HSPA* family gene loci.

Notably, the T7E1 assay demonstrates that the selected population is highly enriched for cells carrying the target mutation. The enrichment factor is approximately 753 (5.5*6.1*3.4*6.6) compared to traditional methods without using a donor. Considering that this calculation does not consider genes with donor insertion, the actual efficiency is even higher.

For puromycin-resistant single clones, PCR verification was performed at five target sites. Specific primers (L5/R5, L6/R6, L7/R7, L8/R8, and L9/R9) used to amplify target sites of all five genes are listed in Table 8. The results are shown in Figure 11a and Figure 11b. Genomic sequence analysis was performed on six selected clones (identified by boxes in the figure and numbered 1-6, respectively) with donor insertions in at least two target genes. The results are shown in Figure 11b. Clone 3 has modifications at the corresponding sites of the four genes: a frameshift mutation in *HSPA1A, HSPA1B* and *HSPA1L*, resulting in a complete knockout, as well as two in-frame mutations in *HSPA6* (Figure 8f).

**Table 8. Primers for Amplifying Five Gene Target Sites of the HSPA Family in HeLaoc Cells**

| Primer pair | Sequence |
|---|---|
| L5/R5 | 5'-GAGAGTGACTCCCGTTGTCC-3'/5'- ACATTGCAAACACAGGAAATTGAG-3' |
| L6/R6 | 5'-GTGTTGAGTTTCCGGCGTTC-3'/5'-TCGCTTGTTCTGGCTGATGT-3' |
| L7/R7 | 5'-GCACTCTCCCAAAACAGTATCTTA-3'/5'- GTGCCTCCACCCAGATCAAA-3' |
| L8/R8 | 5'-GGGTGAGGCGCAAAAGGATA-3'/5'-ACACCAGCGTCAATGGAGAG-3' |

The materials and methods used in Examples 1-7 above were as follows:

### Cell Culture and Transfection

HeLa, HeLaOC and HEK293T cells were maintained in Dulbecco's modified Eagle's medium (DMEM, 10-013-CV, Corning, Tewksbury, MA, USA) supplemented with 10% fetal bovine serum (FBS, Lanzhou Bailing Biotechnology Co., Ltd., Lanzhou, China) at a temperature of 37°C, and bubbled with 5% CO₂. For transfection, all cells were seeded on a 6-well plate and transfected with X-tremeGENE HP (06366546001, Roche, Mannheim, Germany) according to the supplier's instructions. Briefly, 2 µg of DNA and 4 µl of X-tremeGENE HP were added to 200 µl of Opti-MEM I Reduced Serum Medium (31985088, Thermo Fisher Scientific, Grand Island, NY, USA). The mixture was incubated for 15 minutes at room temperature and then added to the cells.

### Cloning of Plasmids Expressing gRNA

For plasmids expressing sgRNAs, the oligonucleotide of each sgRNA encoding sequence was designed separately (see Table 9) and synthesized (Beijing RuiboXingke Biotechnology Co., Ltd.).

**Table 9. Primers for sgRNA or pgRNA Construction**

| sgRNA | Forward primer | Reverse primer |
|---|---|---|
| sgRNA1_{ANTXR1} | 5'-ACCGAGCGGAGAGCCCTCGGC AT-3' | 5'-AAACATGCCGAGGGCTCTCCGC T-3' |
| sgRNA2_{ANTXR1} | 5'-ACCGTGCTCATCTGCGCCGGGC AA-3' | 5'-AAACTTGCCCGGCGCAGATGA GCA-3' |
| pgRNA_{ANTXR1} | 5'-TATACGTCTCAACCGAGCGGAG AGCCCTC GGCATGTTTAAGAGCTATGCTGGA AACAG-3' | 5'-TATACGTCTCAAAACTTGCCCG GCGCAGA TGAGCACGGTGTTTCGTCCTTTCC ACA-3' |
| sgRNA1_{HBEGF} | 5'-ACCGGACTGGCGAGAGCCTGG | 5'-AAACCTCCAGGCTCTCGCCAGT |
| | AG-3' | C-3' |
| sgRNA2_{HBEGF} | 5'-ACCGCGGACCAGCTGCTACCCC T-3' | 5'-AAACAGGGGTAGCAGCTGGTC CG-3' |
| pgRNA_{HBEGF} | 5'-TATACGTCTCAACCGGACTGGC GAGAGCC TGGAGGTTTAAGAGCTATGCTGGA AACAG-3' | 5'-TATACGTCTCAAAACAGGGGTA GCAGCT GGTCCGCGGTGTTTCGTCCTTTCC ACA-3' |
| sgRNA_{PSEN1} | 5'-ACCGCCAGAATGCACAGATGTC TG-3' | 5'-AAACCAGACATCTGTGCATTCT GG-3' |
| sgRNA_{PSEN2} | 5'-ACCGTTCATGGCCTCTGACAGC G-3' | 5'-AAACCGCTGTCAGAGGCCATG AA-3' |
| pgRNA_{PSEN} | 5'-TATACGTCTCaACCGCCAGAATG CACAGAT GTCTGGTTTAAGAGCTATGCTGGA AACA-3' | 5'-TATACGTCTCaAAACCGCTGTCA GAGGC CATGAACGGTGTTTCGTCCTTTCC ACA-3' |
| sgRNA_{HSPA} | 5'-ACCGCAGGAGTAGGTGGTGCC C-3' | 5'-AAACGGGCACCACCTACTCCTG -3' |

Oligonucleotides were dissolved to a concentration of 10 µM with 1x TE, and the paired oligonucleotides were mixed with TransTaq HiFi Buffer II (K10222, Beijing TransGen Biotech Co., Ltd.), heated to 95°C for 3 minutes, and then slowly cooled to 4°C. These annealed oligonucleotide pairs were phosphorylated for 30 minutes at 37°C. After heat inactivation, the product was ligated into the sgRNA backbone vector using the "Golden Gate" method. For plasmids expressing pgRNAs, the scaffold sequence of the gRNA and the U6 promoter were amplified with primers containing two gRNA encoding sequences (Table 5), and the PCR product was then purified and ligated into the sgRNA backbone vector using the "Golden Gate" method. Compared to the previously reported sgRNA backbone vector ^{[17]}, the sgRNA backbone vector of the present invention modified the sgRNA backbone ^{[38]}, and replaced the EGFP sequence with the mCherry encoding sequence.

### T7E1 Assay

Genomic DNA was extracted using a DNeasy Blood & Tissue kit (69504, Qiagen, Hilden, Germany), and the genomic region containing the gRNA target sequence was subjected to PCR amplification. The primer sequences used in the assay are shown in Table 2, Table 5, Table 7, and Table 8. The used primer sequence such as. 300-500 ng of PCR product was mixed with 10x NEB Buffer2 in a 50 µl of system and heated at 95°C for 3 minutes before slowly cooling to room temperature. The resulting product was incubated with 0.5 µl of T7E1 for 15 min at 37°C for agarose gel electrophoresis. The electropherogram was analyzed by Image J image analysis software for the band cleavage efficiency, indicating the production efficiency of Indels by sgRNAs.

### Construction of Linear Donors

The donor and termination codon sequences containing a CMV-driven puromycin-resistant gene or EGFP gene were pre-produced and cloned into the pEASY-T5-Zero clone vector (CT501-02, Beijing TransGen Biotech Co., Ltd.) as a universal template. The template was amplified using primers containing sgRNA cleavage target sites and protection sequences. The primer sequences are shown in Table 10.

**Table 10. Primers for Linear Donor Construction**

| Donor | Primer (forward/reverse) | |
|---|---|---|
| | Step 1 | Step 2 |
| Donor_{ANTXR1-sg1} | 5'-AGAGCCCTCGGCATCGGCTTC CAG TGGCTCTCTTTGGTTAGTCACCT ACTA GTTAGTCA-3'/5'-GGCTTAGGATTG TTAC GCCCTCACTTATCTACTAATCAAT TA-3' | 5'-TCCACTGCGACGTCGCGAGTAG CGGAG AGCCCTCGGCATCGGCTTCCAGTG GCTCTC-3' /5'-GGCTTAGGATTGTTACGCCCTC ACTTATC TACTAATCAATTA-3' |
| Donor_{ANTXR1-sg2} | 5'-TCTGCGCCGGGCAAGGGGGAC GCAGG GAGGATGGGGGTTAGTCACCTA CTAGT TAGTCA-3'/5'-GGCTTAGGATTGT TACGC CCTCACTTATCTACTAATCAATT A-3' | 5'-TCCACTGCGACGTCGCGAGTTG CTCATC TGCGCCGGGCAAGGGGGACGCAG GGAG GAT-3'/5'-GGCTTAGGATTGTTACG CCCTC ACTTATCTACTAATCAATTA-3' |
| Donor_{ANTXR1-pgRNA} | 5'-AGAGCCCTCGGCATCGGCTTC CAGTGG CTCTCTTTGGTTAGTCACCTACT AGTTAG TCA-3'/5'-CCCGGCGCAGATGAGC ACCAG AGTGGCCAAAGAGAGCTCACTT ATCTACT AATCAATTA-3' | 5'-TCCACTGCGACGTCGCGAGTAG CGGAGAG CCCTCGGCATCGGCTTCCAGTGGC TCTC-3' /5'-GGCTTAGGATTGTTACGCCCCC CTTGCC CGGCGCAGATGAGCACCAGAGTG G-3' |
| Donor_{HBEGF-sg1} | 5'-GCGAGAGCCTGGAGCGGCTTC GGAGA GGGCTAGCTGCTTAGTCACCTAC TAGTTA GTCA-3'/5'-GGCTTAGGATTGTTA CGCCC TCACTTATCTACTAATCAATTA-3' | 5'-TCCACTGCGACGTCGCGAGTGA CTGGCGA GAGCCTGGAGCGGCTTCGGAGAG GGCT-3' /5'-GGCTTAGGATTGTTACGCCC TCACTTATCTACTAATCAATTA-3' |
| Donor_{HBEGF-sg2} | 5'-AGCTGCTACCCCTAGGAGGCG GCCGG GACCGGAAAGTTAGTCACCTAC TAGTTAG TCA-3'/5'-GGCTTAGGATTGTTAC GCCCT CACTTATCTACTAATCAATTA-3' | 5'-TCCACTGCGACGTCGCGAGTCG GACCAGCT GCTACCCCTAGGAGGCGGCCGGG ACCGGA-3' /5'-GGCTTAGGATTGTTACGCCCT CACTTATCTACTAATCAATTA-3' |
| Donor_{HBEGF-pgRNA} | 5'-GCGAGAGCCTGGAGCGGCTTC GGAGA GGGCTAGCTGCTTAGTCACCTAC TAGTT AGTCA-3'/5'-GGGTAGCAGCTGGT CCGTG GATACAGTGGGAGGGTCCTCAC TTATCT ACTAATCAATTA-3' | 5'-TCCACTGCGACGTCGCGAGTGA CTGGCGA GAGCCTGGAGCGGCTTCGGAGAG GGCT-3' /5'-GGCTTAGGATTGTTACGCCCCC TAGGGG TAGCAGCTGGTCCGTGGATACAG TGGGA-3' |
| Donor_{PSEN1} | 5'-GATGTCTGAGGACAACCACCT GAGCAA TACTTTAGTCACCTACTAGTTAG TCA-3' /5'-GGCTTAGGATTGTTACGCCCT CACT TATCTACTAATCAATTA-3' | 5'-TCCACTGCGACGTCGCGAGTCC AGAATG CACAGATGTCTGAGGACAACCAC CTG-3' /5'-GGCTTAGGATTGTTACGCCCTC ACTTA TCTACTAATCAATTA-3' |
| Donor_{PSEN2} | 5'-CTGACAGCGAGGAAGAAGTG TGTGAT GAGCGGTTAGTCACCTACTAGTT AGTCA-3' /5'-GGCTTAGGATTGTTACGCCCT CACT TATCTACTAATCAATTA-3' | 5'-TCCACTGCGACGTCGCGAGTTT CATGGCC TCTGACAGCGAGGAAGAAGTG-3' /5'-GGCTTAGGATTGTTACGCCCTC ACTTA TCTACTAATCAATTA-3' |
| Donor_{PSEN1} | 5'-GATGTCTGAGGACAACCACCT GAGCAA TACTTTAGTCACCTACTAGTTAG TCA-3' /5'-GGCTTAGGATTGTTACGCCCC CTCGCT GTCAGAGGCCATGAATGTGAGC ATAGCC-3' | 5'-TCCACTGCGACGTCGCGAGTCC AGAATG CACAGATGTCTGAGGACAACCAC CTG-3' /5'-GCCATGAATGTGAGCATAGCC CTGCCT CTCACTTATCTACTAATCAATTA-3 ' |
| Donor_{HSPA} | 5'-ACCACCTACTCCTGCGTGGGG GTGTTCC AACACGTTAGTCACCTACTAGTT AGTCA-3' /5'-GGCTTAGGATTGTTACGCCCT CACT TATCTACTAATCAATTA-3' | 5'-TCCACTGCGACGTCGCGAGTCC TGGGCA CCACCTACTCCTGCGTGGGGTGT TC-3' /5'-GGCTTAGGATTGTTACGCCCTC ACTTA TCTACTAATCAATTA-3' |

### NHEJ-based Donor Insertion and Cell Selection

HeLaoc cells were transfected with 1 µg of purified linear donor PCR product and 1 µg of sgRNA/pgRNA, and treated with 1 µg/ml puromycin two weeks after transfection. HeLa and HEK293T cells were transfected with 1 µg of donor, 0.5 µg of sgRNA/pgRNA and 0.5 µg of Cas9 plasmid. The cells were then treated with 1 µg/ml puromycin two weeks after transfection, or determined to be EGFP positive by fluorescence activated cell sorting (FACS), depending on which type of donor was used.

### Splinkerette PCR

The Splinkerette PCR method has been previously reported (Potter, C.J. & Luo, L. Splinkerette PCR for mapping transposable elements in Drosophila. PLoS One 5, e10168 (2010); Uren, A.G et al. A high-throughput Splinkerette-PCR method for the isolation and sequencing of retroviral insertion sites. Nat Protoc 4,789-798 (2009); and Yin, B. & Largaespada, D.A. PCR-based procedures to isolate insertion sites of DNA elements. Biotechniques 43, 79-84 (2007)). The primer and adaptor sequences used are shown in Table 11.

**Table 11. Primers for Splinkerette PCR**

| Primer | Sequence |
|---|---|
| long-chain adaptor | 5'-CGAAGAGTAACCGTTGCTAGGAGAGACCGTGGCTGAATGAGACTGGT GTCGA CACTAGTGG-3' |
| short-chain adaptor | 5'-CGCGCCACTAGTGTCGACACCAGTCTCTAATTTTTTTTTCAAAAAAA- 3' |
| Splink1 | 5'-CGAAGAGTAACCGTTGCTAGGAGAGACC-3" |
| Splink2 | 5'-GTGGCTGAATGAGACTGGTGTCGAC-3' |
| R1 | 5'-GCAACCTCCCCTTCTACGAGCGG-3' |
| R2 | 5'-GCATGGCCGTGTTGAGCGGTTCCC-3' |

### References

1. Kim, Y.G., Cha, J. & Chandrasegaran, S. Hybrid restriction enzymes: zinc finger fusions to Fok I cleavage domain. Proc Natl Acad Sci U S A 93, 1156-1160 (1996).
2. Boch, J. et al. Breaking the code of DNA binding specificity of TAL-type III effectors. Science 326, 1509-1512 (2009).
3. Moscou, M.J. & Bogdanove, A.J. A simple cipher governs DNA recognition by TAL effectors. Science 326, 1501 (2009).
4. Miller, J.C. et al. A TALE nuclease architecture for efficient genome editing. Nat Biotechnol 29, 143-148 (2011).
5. Jinek, M. et al. A programmable dual-RNA-guided DNA endonuclease in adaptive bacterial immunity. Science 337, 816-821 (2012).
6. Mali, P. et al. RNA-guided human genome engineering via Cas9. Science 339, 823-826 (2013).
7. Cong, L. et al. Multiplex Genome Engineering Using CRISPR/Cas Systems. Science 339, 819-823 (2013).
8. Phillips, E.R. & McKinnon, P.J. DNA double-strand break repair and development. Oncogene 26, 7799-7808 (2007).
9. Chapman, J.R., Taylor, M.R. & Boulton, S.J. Playing the end game: DNA double-strand break repair pathway choice. Mol Cell 47, 497-510 (2012).
10. Gilbert, L.A. et al. CRISPR-mediated modular RNA-guided regulation of transcription in eukaryotes. Cell 154, 442-451 (2013).
11. Ma, H. et al. Multicolor CRISPR labeling of chromosomal loci in human cells. Proc Natl Acad Sci U S A 112, 3002-3007 (2015).
12. Chen, B. et al. Dynamic imaging of genomic loci in living human cells by an optimized CRISPR/Cas system. Cell 155, 1479-1491 (2013).
13. Li, H.L., Gee, P., Ishida, K. & Hotta, A. Efficient genomic correction methods in human iPS cells using CRISPR-Cas9 system Methods (2015).
14. Savic, N. & Schwank, G. Advances in therapeutic CRISPR/Cas9 genome editing. Translational research : the journal of laboratory and clinical medicine 168, 15-21 (2016).
15. Miyaoka, Y. et al. Isolation of single-base genome-edited human iPS cells without antibiotic selection. Nat Methods 11, 291-293 (2014).
16. Fu, Y. et al. High-frequency off-target mutagenesis induced by CRISPR-Cas nucleases in human cells. Nat Biotechnol 31, 822-826 (2013).
17. Zhou, Y. et al. High-throughput screening of a CRISPR/Cas9 library for functional genomics in human cells. Nature 509, 487-491 (2014).
18. Yu, C. et al. Small Molecules Enhance CRISPR Genome Editing in Pluripotent Stem Cells. Cell Stem Cell 16, 142-147 (2015).
19. Liao, S., Tammaro, M. & Yan, H. Enriching CRISPR-Cas9 targeted cells by co-targeting the HPRT gene. Nucleic Acids Res 43, e134 (2015).
20. Kim, H. et al. Surrogate reporters for enrichment of cells with nuclease-induced mutations. Nat Methods 8, 941-943 (2011).
21. Ramakrishna, S. et al. Surrogate reporter-based enrichment of cells containing RNA-guided Cas9 nuclease-induced mutations. Nature communications 5, 3378 (2014).
22. Wang, T., Wei, J.J., Sabatini, D.M. & Lander, E.S. Genetic screens in human cells using the CRISPR-Cas9 system. Science 343, 80-84 (2014).
23. Yuan, P. et al. Chondroitin sulfate proteoglycan 4 functions as the cellular receptor for Clostridium difficile toxin B. Cell Res 25, 157-168 (2015).
24. Yang, J. et al. ULtiMATE System for Rapid Assembly of Customized TAL Effectors. PLoS One 8, e75649 (2013).
25. Perez, E.E. et al. Establishment of HIV-1 resistance in CD4+ T cells by genome editing using zinc-finger nucleases. Nat Biotechnol 26, 808-816 (2008).
26. Kim, H.J., Lee, H.J., Kim, H., Cho, S.W. & Kim, J.S. Targeted genome editing in human cells with zinc finger nucleases constructed via modular assembly. Genome Res 19, 1279-1288 (2009),
27. Lackner, D.H. et al. A generic strategy for CRISPR-Cas9-mediated gene tagging. Nature communications 6, 10237 (2015).
28. Auer, T.O. & Del Bene, F. CRISPR/Cas9 and TALEN-mediated knock-in approaches in zebrafish Methods (2014)
29. Li, K., Wang, G., Andersen, T., Zhou, P. & Pu, W.T. Optimization of Genome Engineering Approaches with the CRISPR/Cas9 System. PLoS One 9, e105779 (2014).
30. Orlando, S.J. et al. Zinc-finger nuclease-driven targeted integration into mammalian genomes using donors with limited chromosomal homology. Nucleic Acids Res 38, e152 (2010).
31. Sakuma, T., Nakade, S., Sakane, Y., Suzuki, K.T. & Yamamoto, T. MMEJ-assisted gene knock-in using TALENs and CRISPR-Cas9 with the PITCh systems. Nat Protoc 11, 118-133 (2016).
32. Nakade, S. et al. Microhomology-mediated end-joining-dependent integration of donor DNA in cells and animals using TALENs and CRISPR/Cas9. Nature communications 5, 5560 (2014).
33. Cristea, S. et al. In vivo cleavage of transgene donors promotes nuclease-mediated targeted integration. Biotechnol Bioeng 110, 871-880 (2013).
34. Chen, F. et al. High-frequency genome editing using ssDNA oligonucleotides with zinc-finger nucleases. Nat Methods 8, 753-755 (2011).
35. Potter, C.J. & Luo, L. Splinkerette PCR for mapping transposable elements in Drosophila. PLoS One 5, e10168 (2010).
36. Uren, A.G. et al. A high-throughput splinkerette-PCR method for the isolation and sequencing of retroviral insertion sites, Nat Protoc 4, 789-798 (2009).
37. Yin, B. & Largaespada, D.A. PCR-based procedures to isolate insertion sites of DNA elements. Biotechniques 43, 79-84 (2007).
38. Peng, J., Zhou, Y., Zhu, S. & Wei, W. High-throughput screens in mammalian cells using the CRISPR-Cas9 system. FEBS J 282, 2089-2096 (2015).

## Claims

1. A donor construct, which is a linear donor DNA or capable of producing a linear donor DNA by cleavage in a cell, wherein the linear donor DNA sequentially comprises, from the middle to both ends: an expression cassette; a short sequence extension consisting of reverse termination codons which is located at the 5'-end of the expression cassette and a short sequence extension consisting of forward termination codons which is located at the 3'-end of the expression cassette; a target sequence located at the 5'-end and/or 3'-end, the target sequence comprising a target site capable of being cleaved by a sequence-specific nuclease; and protection sequences located at both ends; and wherein the expression cassette comprises a promoter-driven marker gene;
preferably, the sequence-specific nuclease is a zinc finger nuclease (ZFN), a transcription activator-like effector nuclease (TALEN), a Cas9 nuclease, or an NgAgo nuclease;
preferably, the linear donor DNA has a target sequence only at the 5'-end or 3'-end, or alternatively, the linear donor DNA has target sequences at both ends, respectively; further preferably, the target sequences at both ends of the linear donor DNA are different; still further preferably, the different target sequences at both ends of the linear donor DNA are derived from the same gene, or alternatively, the different target sequences at both ends of the linear donor DNA are derived from different genes; and
preferably, the marker gene is an antibiotic resistance gene or a fluorescent protein gene.

2. A method of generating a gene knockout in a cell, comprising the steps of:
(1) introducing into a cell a sequence-specific nuclease capable of cleaving a specific target site in the cell genome and a donor construct;
wherein the donor construct is a linear donor DNA or capable of producing a linear donor DNA by cleavage in a cell, wherein the linear donor DNA sequentially comprises, from the middle to both ends: an expression cassette; a short sequence extension consisting of reverse termination codons which is located at the 5'-end of the expression cassette and a short sequence extension consisting of forward termination codons which is located at the 3'-end of the expression cassette; a target sequence located at the 5'-end and/or 3'-end, the target sequence comprising a target site capable of being cleaved by a sequence-specific nuclease; and protection sequences located at both ends; and wherein the expression cassette comprises a promoter-driven marker gene; and
wherein the linear donor DNA is inserted into the specific target site in the cell genome by non-homologous end joining; and
(2) screening cells positive for the marker expression;
preferably, the gene knockout is a single gene knockout or a multi-gene knockout; and
preferably, the marker gene is an antibiotic resistance gene or a fluorescent protein gene; further preferably, the cells are screened by drug resistance in step (2), or alternatively, the cells are screened by the FACS method in step (2).

3. The method of claim 2, wherein the sequence-specific nuclease is a zinc finger nuclease (ZFN), or a transcription activator-like effector nuclease (TALEN).

4. The method of claim 2, wherein the sequence-specific nuclease is a Cas9 nuclease; and
preferably, step (1) further comprises introducing into a cell an sgRNA that recognizes a specific target site in the cell genome, wherein the target sequence in the linear donor DNA comprises a target site recognized by the sgRNA.

5. The method of claim 4, wherein the sgRNA recognizes a single specific target site in the cell genome, and the target sequence comprising the target site recognized by the gRNA is located at the 5'-end and/or 3'-end of the linear donor DNA; and
preferably, the target sequence comprising the target site recognized by the gRNA is derived from a single gene in the cell genome; or alternatively, the target sequence comprising the target site recognized by the gRNA is the consensus sequence of two or more genes in the cell genome, provided that the consensus sequence has no more than one base difference from the sequence at positions corresponding to the consensus sequence in any of the two or more genes.

6. The method of claim 4, wherein the sgRNA is two sgRNAs that respectively recognize two specific target sites on one gene in the cell genome; and two target sequences respectively comprising the two target sites recognized by the two sgRNAs are located in two linear donor DNAs, respectively, or located at both ends of the same linear donor DNA, respectively.

7. The method of claim 4, wherein the sgRNA is two or more sgRNAs; two or more target sequences respectively comprising two or more target sites recognized by the two or more sgRNAs are located at both ends of the same linear donor DNA, respectively, or located in different linear donor DNAs; and the two or more target sites are located on different genes in the cell genome, respectively.

8. The method of claim 2, wherein the sequence-specific nuclease is an NgAgo nuclease; and
preferably, step (1) further comprises introducing into a cell a gDNA that recognizes a specific target site in the cell genome, wherein the target sequence in the linear donor DNA comprises a target site recognized by the gDNA.

9. A system for gene knockout, comprising: a sequence-specific nuclease capable of cleaving a specific target site in the cell genome and a donor construct;
wherein the donor construct is a linear donor DNA or capable of producing a linear donor DNA by cleavage in a cell, wherein the linear donor DNA sequentially comprises, from the middle to both ends: an expression cassette; a short sequence extension consisting of reverse termination codons which is located at the 5'-end of the expression cassette and a short sequence extension consisting of forward termination codons which is located at the 3'-end of the expression cassette; a target sequence located at the 5'-end and/or 3'-end, comprising a target site capable of being cleaved by a sequence-specific nuclease; and protection sequences located at both ends; and wherein the expression cassette comprises a promoter-driven marker gene; and
preferably, the donor construct is a circular donor construct and is capable of producing a linear donor DNA by cleavage in a cell;
preferably, the sequence-specific nuclease is a zinc finger nuclease (ZFN), or a transcription activator-like effector nuclease (TALEN);
preferably, the sequence-specific nuclease is a Cas9 nuclease; further preferably, the system further comprises an sgRNA that recognizes a specific target site in the cell genome, wherein the target sequence in the linear donor DNA comprises a target site recognized by the sgRNA;
preferably, the sequence-specific nuclease is an NgAgo nuclease; further preferably, the system or kit further comprises a gDNA that recognizes a specific target site in the cell genome, wherein the target sequence in the linear donor DNA comprises a target site recognized by the gDNA; and
preferably, the marker gene is an antibiotic resistance gene or a fluorescent protein gene.

10. A kit consisting of the system of claim 9, for use in gene knockout.
